# EUROPÄISCHE PATENTSCHRIFT

(11) **EP 1 637 882 B1**
(45) Veröffentlichungstag und Bekanntmachung des Hinweises auf die Patenterteilung: **08.08.2007**
(21) Anmeldenummer: 04021433.0
(22) Anmeldetag: 09.09.2004
(51) Int. Cl.: G01N 33/543, C12Q 1/00

(54) **Lateralflussmessvorrichtung und Messverfahren für Analyten**
Lateral-flow measuring device and method for the measurement of analytes
Dispositif a écoulement latéral et méthode pour mesurer des analytes

(43) Veröffentlichungstag der Anmeldung: 22.03.2006
(73) Patentinhaber: Analyticon Biotechnologies AG, 35104 Lichtenfels (DE)
(72) Erfinder: Meisegeier, Bernhard, Dr., 97209 Veithöchheim (DE); Oberstrass, Jürgen, Dr., 34317 Habichtswald (DE); Zwingmann, Detlev, Dr., 35037 Marburg (DE); Schaper, Reinhard, Dr., 34497 Korbach (DE)
(74) Vertreter: Buchhold, Jürgen

(56) Entgegenhaltungen:
- GB-A- 2 391 068
- US-A1- 2003 180 815
- US-A1- 2004 106 190
- US-B1- 6 197 494
- US-B1- 6 670 115

## Beschreibung

Die Erfindung betrifft eine Meßvorrichtung zum Erfassen und/oder zur Bestimmung wenigstens eines in einem flüssigen Medium gelösten Analyten gemäß dem Oberbegriff von Anspruch 1, ein Verfahren zum Herstellen der Meßvorrichtung gemäß dem Oberbegriff von Anspruch 39, ein Verfahren zu deren Bedienung gemäß dem Oberbegriff von Anspruch 44 und eine Verwendung der Meßvorrichtung gemäß Anspruch 49.

Sensoren zur Bestimmung von Analyten in einer Probenlösung sind im Stand der Technik bekannt. So hat man in DE-A1-100 32 042 ein System aus einer Arbeitselektrode und einer Gegenelektrode auf einem Kunststoff-Trägermaterial aufgebracht. Die Arbeitselektrode ist mehrschichtig aufgebaut, nämlich wenigstens aus einer Reaktionsschicht und aus einer Schutzschicht. Die Schutzschicht ist erforderlich, weil elektrochemische Signale in einer Probenlösung auf einen sublimierbaren Mediator und von dort auf die Arbeitselektrode übertragen werden. Ohne die Schutzschicht würde der Mediator absublimieren und den Sensor in seiner Funktion beeinträchtigen. Die Mehrschichtanordnung erschwert es jedoch, eine genau definierte Menge an Proben- oder Substratlösung mit der Arbeitselektrode in Kontakt zu bringen, was zu Meßungenauigkeiten führen kann. Außerdem verlangt eine Messung mehrere Waschschritte und verlängert die Meßzeit.

In der WO-A1-99/58709 wird eine Testvorrichtung beschrieben, die aus einem Träger, einer darauf aufliegenden Elektrodenanordnung aus drei unterschiedlich dimensionierten Elektroden und aus mehreren darauf abgelegten netzartigen Geweben und Deckschichten besteht. Diese Schichten und Gewebe isolieren die einzelnen Elektroden gegeneinander. Außerdem sorgen sie dafür, daß eine durch eine Öffnung in der obersten Schicht aufgetragene Meßprobe sich mehr oder weniger gut durch den flexiblen Schichtstapel um die Elektroden herum verteilt. Der elastische Stapel wird nacheinander aufgetragene Flüssigkeitsproben jedoch nicht jedesmal genau in der gleichen Weise aufnehmen und zwischen den einzelnen Schichten verteilen. Deshalb kann sich keine konstante Menge an Meßprobe in einem definierten Volumenbereich um die Elektroden herum anordnen. Dadurch können Meßfehler auftreten und Messungen schwer reproduzierbar werden. Im übrigen ist die beschriebene sandwichartige Stapelanordnung aufwendig in der Herstellung.

Die Nachteile einer flexiblen Bauweise überwindet die in der EP-B1-0 170 375 beschriebene Testvorrichtung. Sie offenbart eine Meßzelle, bestehend aus zwei plattenförmigen Hartkunststoffflächen. Diese sind zueinander eng beabstandet angeordnet und schließen eine teilchenselektive Elektrodenanordnung zwischen sich ein. Der Abstand beider Flächen zueinander wird durch Grate erreicht, die entlang der Flächenlängsseiten verlaufen. Eine der beiden Kunststoffflächen ist größer ausgebildet als die andere und bildet eine Lippe aus, auf die eine Analysenlösung aufgetragen werden kann. Diese strömt durch Kapillarkräfte getrieben bis an die Elektrodenanordnung und verdrängt dabei vor ihr befindliche Luft stromabwärts aus der Testvorrichtung heraus. Demnach wird solange Analysenlösung in die Testvorrichtung einströmen, bis letztere vollständig mit Flüssigkeit gefüllt ist. Es gelingt nicht, darüber hinaus weiter Flüssigkeit, beispielsweise zu Waschzwecken, in das Innere der Testvorrichtung hineinzubringen, ohne vorher das bereits zwischen den beiden Platten eingeschlossene Lösungsvolumen mittels eines saugfähigen Vlieses oder Gewebes aufwendig zu entfernen.

Auch die WO-A1-00/70327 vermag die genannten Nachteile nicht vollständig zu überwinden. Sie offenbart ein Testgerät für die quantitative elektrochemische Analyse eines Analyten in einer Festphase. Es besteht aus einer Schicht mit Elektroden auf der eine Stützschicht mit einem Loch aufgebracht ist. Dieses liegt unmittelbar über den Elektroden. Auf der Stützschicht liegt ein chromatographischer Teststreifen auf. Dieser ist dreigeteilt und besteht aus einem Probensammelkissen, einer sich daran anschließenden als Membran bezeichneten Fließfläche und einem Absorptionskissen. Das Probensammelkissen enthält eine lösliche für einen Analyten spezifische Substanz, die eine Markierung trägt.

In einem ersten Schritt trägt man eine flüssige Probe auf das Probensammelkissen auf. Ein Großteil der Probenflüssigkeit fließt über die Membran in das Absorptionskissen. Nur ein kleiner Teil gelangt in das Loch über den Elektroden. Dieses wird sich deshalb nur bei großen Probenvolumina vollständig mit Flüssigkeit füllen können. Ist der gesuchte Analyt in der stromabwärts fließenden Probe enthalten, bildet er mit der spezifischen Substanz einen Komplex. Dieser wandert weiter stromabwärts und bindet an eine weitere auf der Membran im Bereich des Lochs immobilisierte spezifische Substanz. Es entsteht eine Sandwich-Verbindung, deren Menge nur dann elektrochemisch detektiert werden kann, wenn die Membran und die Elektroden miteinander über das Loch in flüssigem Kontakt stehen.

In einem zweiten Schritt trägt man eine Substratlösung entweder auf das Probensammelkissen oder unmittelbar auf die Membran oberhalb des Lochs auf. Hierdurch füllt sich letzteres je nach aufgebrachter Menge mehr oder weniger vollständig mit Flüssigkeit. Diese vermischt sich mit der dort schon vorhandenen Probenlösung. Es gelingt also nicht, eine definierte Menge einer einzigen Lösung im Loch zu plazieren, die zudem frei von Spuren der jeweils anderen Lösung ist. Außerdem ist es nicht möglich, die Probenlösung vollständig gegen die Substratlösung auszutauschen. Dies kann zu Messungenauigkeiten und nicht reproduzierbaren Meßergebnissen führen, insbesondere, wenn die Probenlösung elektroaktive Bestandteile enthält, die in der Substratlösung nicht vorhanden sind. Im übrigen ist das aus vielen Einzelteilen bestehende Testgerät in seiner Architektur aufwendig und damit kostspielig herzustellen.

Die US-A1-2003/0 180 815 beschreibt ein Testgerät aus einem Spritzguß-Kunststoffgehäuse, in dem sich ein Teststreifen mit einem Probenkissen und einer Fangzone befindet. Das Probenkissen enthält einen beweglichen mit einem Enzym markierten analytispezifischen Antikörper und die Fangzone einen zweiten ebenfalls analytspezifischen Fängerantikörper sowie ein Absorptionskissen, welches sich mit einer Elektrode in Kontakt bringen läßt. Wird eine Messprobe auf das Probenkissen aufgegeben, wandert sie in Richtung des Absorptionskissens und der in ihr evtl. vorhandene Analyt bindet an den Fängerantikörper. Wieviel Analyt gebunden wird, hängt von der aufgetragenen Probenmenge, der Position des Probenauftrags, sowie der Saugwirkung von Probenkissen und Absorptionskissen ab. Diese Bedingungen lassen sich nur schwer reproduzieren und deshalb kann es zu Messungenauigkeiten kommen.

Gegenstand der US-A1-2004 / 0 106 190 ist eine Vorrichtung zum Erfassen wenigstens eines in einem flüssigen Medium vorhandenen Analyten. Sie umfasst einen streifenförmigen Träger, auf dem ein gedruckter Mehrfach-Elektrodensatz aus wenigstens einer Referenzelektrode und aus wenigstens einer Arbeitselektrode aufgebracht ist. Ferner besitzt sie ein stromaufwärts angeordnetes Konjugatkissen. In einer Ausführungsform ist anstelle dieses Kissens eine Kammer vorgesehen, von der Flüssigkeit durch einen Kanal in eine Detektionszone strömt. Kammer und Kanal können vielfältigste Formen annehmen sind jedoch für die Aufnahme von Messprobe nach oben offen. Weil Probe sich aufgrund dieser vielfältigen Formenwahl in unterschiedlicher nicht reproduzierbarer Weise in der Testvorrichtung verteilen kann, wird immer eine unterschiedliche Menge an Analyt den Elektrodensatz erreichen, was zu Messfehlern führen kann.

Die US-B1-6,670,115 offenbart eine Vorrichtung zum Erfassen und/oder zur Bestimmung der Konzentration wenigstens eines in einem flüssigen Medium vorhandenen Analyten, bestehend aus einem streifenförmigen Träger mit zwei Längskanten, auf dem ein gedruckter Mehrfach-Elektrodensatz aus wenigstens einer Referenzelektrode und aus wenigstens einer Arbeitselektrode aufgebracht ist. Jede Elektrode ist zum Anschluß an eine Auslesevorrichtung mit einer Leiterbahn versehen und auf der Arbeitselektrode ist Bindungsreagenz, das an einen gesuchten Analyten binden kann, unlösbar aufgebracht. Eine auf einem Probenkissen vorgesehene Probenauftragszone ist vom Mehrfachelektrodensatz beabstandet angeordnet, steht mit ihm jedoch über ein Saugkissen in Kontakt, sobald das flüssige Medium den Träger durchfließt. Dieses Saugkissen besitzt eine offene Netzstruktur und soll einen schnelleren lateralen Probendurchfluß bewirken, als ein Saugkissen mit einer geschlossenen Netzstruktur. Es ist an einer Seite mit dem Probenkissen und an der anderen Seite mit einem Konjugatkissen sowie mit dem Mehrfachelektrodensatz in Berührungsverbindung.

Zur Bestimmung eines Analyten gibt man Messprobe auf die Probenauftragszone. Von dort verteilt sie sich anteilig im Probenkissen und im Saugkissen. Der in das Saugkissen strömende Anteil bindet bei Anwesenheit des gesuchten Analyten ein Konjugatreagenz aus dem Konjugatkissen und der dabei entstandene Komplex strömt an die Arbeitselektrode, um sich dort mit dem Bindungsreagenz zu einem Sandwich zu verbinden. Nachteilig bei dieser Anordnung ist, daß sich Messprobe im Proben-, im Saug- und im Konjugatkissen verteilt. Deshalb ist es nicht möglich, eine definierte Probenmenge mit dem Mehrfachelektrodensatz in Kontakt zu bringen, was zu ungenauen Messergebnissen führen kann.

Ziel der Erfindung ist es, unter Überwindung der Nachteile des Standes der Technik eine Meßvorrichtung und ein Verfahren bereitzustellen, um Analytkonzentrationen in flüssigen Medien exakt und schnell nachzuweisen. Dabei sollen elektroaktive Begleitsubstanzen in einer Meßprobe das Meßergebnis genauso wenig beeinflussen wie unterschiedliche Volumina der verwendeten Flüssigkeiten. Die Meßvorrichtung soll unter geringem Materialeinsatz preisgünstig und einfach herzustellen sein und auch nach längerer Lagerung mechanisch und chemisch noch so robust sein, daß sie präzise und reproduzierbare Meßwerte liefert.

Hauptmerkmale der Erfindung sind im kennzeichnenden Teil der Ansprüche 1, 39 und 44 sowie in Anspruch 49 angegeben. Ausgestaltungen sind Gegenstand der Ansprüche 2 bis 38, 40 bis 43 und 45 bis 48.

Bei einer Vorrichtung zum Erfassen und/oder zur Bestimmung der Konzentration wenigstens eines in einem flüssigen Medium vorhandenen Analyten, bestehend aus einem streifenförmigen Träger mit zwei Längskanten, auf dem ein gedruckter Mehrfach-Elektrodensatz aus wenigstens einer Referenzelektrode und aus wenigstens einer Arbeitselektrode aufgebracht ist, ist jede Arbeitselektrode und jede Referenzelektrode mit einer ihr zugeordneten Leiterbahn und jede Leiterbahn mit einem ihr zugeordneten, an ein elektrisches Meßgerät anschließbaren Kontakt leitend verbunden. Ferner umfasst die Vorrichtung Liganden, die geeignet sind, ein biologisch aktives spezifisches Bindungsmolekül oder den Analyten zu binden, sowie eine an einem Ende des Trägers ausgebildete Probenauftragszone.

Erfindungsgemäß ist vorgesehen, daß die Vorrichtung einen starren, kontinuierlich entlang des streifenförmigen Trägers durchströmbaren Reaktions- und Detektionsraum besitzt, der durch eine auf den Längskanten des Trägers aufgebrachte längliche Unterstützung mit einer darauf angeordneten Abdeckung gebildet ist und daß stromabwärts des Reaktions- und Detektionsraums ein Flüssigkeitsaufnahmeelement angeordnet ist.

Zudem ist die längliche Unterstützung zweigeteilt und liegt in Form von zwei Streifen auf den beiden Längskanten des Trägers permanent haftend auf und zwar derart, daß die Streifen nur einen Teil der beiden Längskanten des Trägers bedecken, wobei zwischen den Streifen und dem Flüssigkeitsaufnahmeelement ein Zwischenraum besteht, von dem aus sich die Streifen auf den Längskanten stromaufwärts erstrecken.

Meßlösungen lassen sich in unterschiedlichen Mengen auf den Träger auftragen um diesen in Richtung Flüssigkeitsaufnahmeelement zu durchströmen. Im Reaktions- und Detektionsraum bleibt aber unabhängig vom Ausgangsvolumen stets eine konstante Lösungsmenge zurück, weshalb sehr präzise Messungen durchgeführt werden können. Außerdem lassen sich Lösungsspuren aus dem Reaktions- und Detektionsraum mit einer zweiten Lösung herausspülen. Auch jetzt verbleibt exakt die gleiche Flüssigkeitsmenge im Reaktions- und Detektionsraum. Deshalb lassen sich Analytkonzentrationen auch dann noch sehr präzise bestimmen, wenn für eine Messung mehrere Lösungen nacheinander eingesetzt werden müssen. Der Zwischenraum trägt dazu bei und sorgt dafür, daß bei jedem Flüssigkeitsauftrag auf den Träger eine definierte Probenmenge im Reaktions- und Detektionsraum verbleibt.

Der Träger ist nach Anspruch 2 als selbsttragende Fließfläche für das flüssige Medium ausgebildet. Gemäß Anspruch 3 ist er einstückig und besteht aus einem polymeren, nichtleitenden, vorzugsweise rechteckigen Material. Diese Ausführungsarten des Trägers mindern die Herstellungskosten und reduzieren das Gewicht der Meßvorrichtung. Sollen ausgewählte Moleküle auf der Oberfläche des Trägers binden, bringt man auf ihn nach Anspruch 4 Liganden und/oder biologisch aktive spezifische Bindungsmoleküle auf. Man kann einige von ihnen oder alle gemäß Anspruch 5 auch auf dem Träger immobilisieren. Hierdurch gewinnt man auf den Elektroden Platz, um auf deren Oberflächen noch andere Moleküle zu fixieren.

Verwendet man Trägermaterialien, die sehr reaktionsträge sind bzw. keine Fähigkeit zur Bindung von biologisch aktiven spezifischen Bindungsmolekülen besitzen, muß deren Oberfläche für aufzubringende Liganden vorbereitet werden. Deshalb sieht Anspruch 6 vor, den Träger mit einer Grundbeschichtung auszustatten, die aus Proteinen aus der Gruppe der tierischen oder menschlichen Albumine und/oder aus der Gruppe der Globuline und/oder aus der Gruppe der Glykoproteine besteht. Nach Anspruch 7 können zu diesem Zweck jedoch auch Polypeptide, beispielsweise Polyaminosäuren und/oder Abbauprodukte von Proteinen benutzt werden, die vorzugsweise dem Stütz- und Bindegewebe entstammen.

Anspruch 8 bestimmt, daß die Probenauftragszone oder wenigstens ein Teil von ihr mit grenzflächenaktiven Stoffen behandelt ist und die Fähigkeit besitzt, das flüssige Medium aufzusaugen. Eine noch weitere Verbesserung der Probenaufnahme erzielt man nach Anspruch 9, indem auf der Probenauftragszone ein maschenförmiges oder poröses, vorzugsweise mit grenzflächenaktiven Stoffen behandeltes Material aufgebracht ist.

Als Material für wenigstens eine der Elektroden sowie für die ihr jeweils zugeordnete Leiterbahn und den ihr jeweils zugeordneten Kontakt verwendet man nach Anspruch 10 Gold, Silber, Platin, Nickel, Palladium, Titan, Kupfer oder Kohlenstoff. Besonders günstig ist es, wenn im Einklang mit Anspruch 11 wenigstens eine der Elektroden aus einer getrockneten Graphitpaste besteht oder eine aus Metallpasten und/oder Metallsalzpasten durch Trocknung herstellbare Elektrode ist. Derartige Elektrodenpasten lassen sich in beliebiger Form auf dem Träger verteilen und ermöglichen somit nahezu jede Elektrodengeometrie.

In einer weiteren Ausgestaltung der Erfindung nach Anspruch 12 trägt wenigstens eine Arbeitselektrode Liganden und/oder biologisch aktive spezifische Bindungsmoleküle, beispielsweise an ihrer Oberfläche. Bringt man solche Teilchen direkt an der Elektrode an, entstehen elektroaktive Substratformen unmittelbar an der Elektrodenoberfläche und es kommt zu einer schnellen Signalweiterleitung. Die Elektrodenbeschichtung läßt sich noch ergänzen, wenn auf wenigstens einer weiteren Arbeitselektrode reaktionsunspezifische Bindungsmoleküle im Sinne von Anspruch 13 angeordnet und/oder immobilisiert sind. Derartige Teilchen registrieren Änderungen in der Meßlösung, die nicht durch ein bestimmtes elektroaktives Substrat entstanden sind und sorgen mithin dafür, daß es nicht zu Meßfehlern kommt.

Wie Anspruch 14 zu entnehmen ist, sind die Liganden als Lösung mit einer Konzentration von 0,0007 bis 0,7 mol/l, vorzugsweise von 0,035 bis 0,35 mol/l aufgetragen. Sie bestehen aus unterschiedlichen für die Erfindung wichtigen Verbindungsklassen. So sind sie nach Anspruch 15 aus Ribonukleinsäuren oder Desoxyribonukleinsäuren gebildet oder sie bestehen laut Anspruch 16 aus Aminocarbonsäuren, vorzugsweise Diaminocarbonsäuren. Besonders bedeutsam sind nach Anspruch 17 höhermolekulare, homodet oder heterodet peptidartig verknüpfte Aminocarbonsäuren der Zusammensetzung R-CH(NH₂)-(CH₂)ₙ-COOH, beispielsweise Polyaminocarbonsäuren, wobei der Rest R für ein Proton oder vorzugsweise für eine Amino-, Imino-, Hydroxyl-, Thiol-, Hydroxyalkyl-, Aminoalkyl- oder Carboxyalkyl-Gruppe steht und n einen Wert von 0 bis 6, vorzugsweise 0 annimmt.

Als Liganden lassen sich im Einklang mit Anspruch 18 auch Proteide bzw. Proteine verwenden, die vorzugsweise aus dem Bakterium Streptomyces avidinii isoliert wurden. Schließlich sieht Anspruch 19 beispielsweise für den Nachweis von HbA1c vor, daß wenigstens ein Ligand eine Phenylboronsäure ist.

Die biologisch aktiven spezifischen Bindungsmoleküle besorgen entsprechend ihrem Namen eine für die Erfindung erforderliche Verknüpfung mit Oberflächen, Liganden und Analyten. Sie besitzen zu diesem Zweck gemäß Anspruch 20 Bindungsstellen für wenigstens einen Liganden oder sind an ihn gebunden. Außerdem weisen sie laut Anspruch 21 für die Bindung eines Analyten wenigstens eine spezifische Bindungsregion auf.

Nach Anspruch 22 ist ein oder sind mehrere katalytisch aktive Proteine an die biologisch aktiven spezifischen Bindungsmoleküle und/oder die Liganden vorzugsweise kovalent gebunden. Durch dieses erfindungswesentliche Merkmal kann ein analytbindendes Molekül gleichzeitig ein Substratmolekül in eine elektroaktive Form überführen.

Um zu erreichen, daß der Reaktions- und Detektionsraum ein für die Erfindung wesentliches konstantes Volumen umschließt, müssen dessen Bestandteile definiert sein. Anspruch 23 bestimmt, daß die Streifen aus einem Polymerwerkstoff und/oder aus einem Schmelzkleber bestehen und die gleiche Dicke wie das Flüssigkeitsaufnahmeelement besitzen.

Die Abdeckung unterstützt die Schaffung des konstanten Volumens innerhalb des Reaktions- und Detektionsraums. Sie besteht nach Anspruch 24 aus einem polymeren, vorzugsweise rechteckigen Material und ist mit der länglichen Unterstützung derart verbunden, daß sie den Träger sowie den Mehrfach-Elektrodensatz und das Flüssigkeitsaufnahmeelement bedeckt. Sie kann in einer Weiterführung nach Anspruch 25 auch nur einen oder mehrere Teile des Trägers bedecken.

Im übrigen zeichnet sie sich nach Anspruch 26 dadurch aus, daß sie an ihrer stromaufwärtigen Seite wenigstens eine schlitz- und/oder halbkreisförmige beziehungsweise vieleckige Aussparung und/oder Öffnung besitzt, die so angeordnet ist, daß die Probenauftragszone des Trägers mit flüssigem Medium beschickbar ist. In einer Ausgestaltung der erfindungsmäßigen Vorrichtung nach Anspruch 27 ist das Flüssigkeitsaufnahmeelement an einer dem Träger zugewandten Seite der Abdeckung haftend befestigt.

Die Oberflächeneigenschaften der Abdeckung lassen sich entscheidend verfeinern und verbessern. Nach Anspruch 28 behandelt man sie oder wenigstens einen Teil von ihr dazu mit grenzflächenaktiven Stoffen und verleiht ihr dadurch eine hydrophile und/oder hydrophobe Oberfläche. In einer ausgestalteten Variante nach Anspruch 29 besitzt die dem Träger zugewandte Seite der Abdeckung im Bereich des Mehrfach-Elektrodensatzes, insbesondere oberhalb der Arbeitselektrode(n) und oberhalb der Referenzelektrode hydrophile Oberflächeneigenschaften und weist am Ort der Probenauftragszone zumindest teilweise eine hydrophobe Oberfläche auf.

Der Reaktions- und Detektionsraum ist im Einklang mit Anspruch 30 in stromabwärtiger Richtung und in stromaufwärtiger Richtung offen und der Zwischenraum grenzt stromabwärts an ihn an. Somit erreicht man einerseits einen kontinuierlichen Flüssigkeitsstrom durch den Träger, und fördert bzw. bewirkt andererseits, daß ein konstantes Lösungsvolumen im Reaktions- und Detektionsraum verbleibt.

Das Flüssigkeitsaufnahmeelement ist für die Erfindung unerläßlich, weil durch dessen Saugwirkung eine Meßlösung besser stromabwärts fließen kann. Zu diesem Zweck ist es entsprechend Anspruch 31 auf dem Träger stromabwärts von der Probenauftragszone beabstandet angeordnet und überdeckt die Leiterbahnen, indem es beispielsweise fest auf ihnen aufliegt.

Um das flüssige Medium besonders gut aufzunehmen, besteht das Flüssigkeitsaufnahmeelement laut Anspruch 32 aus einem saugfähigen porösen fibrillären Material und ist auf dem Träger haftend befestigt. Es ist im Einklang mit Anspruch 33 aus wenigstens einer Faserschicht oder aus wenigstens einem Gewebe aufgebaut, welche(s) aus Cellulosefasern oder Glasfasern oder Gemischen daraus besteht bzw. sich aus organischen Polymeren zusammensetzt. Eine praktische Weiterführung der Erfindung sieht gemäß Anspruch 34 vor, daß das Flüssigkeitsaufnahmeelement mit grenzflächenaktiven Stoffen behandelt ist, vorzugsweise mit solchen, die hydrophile Eigenschaften haben. Dies erhöht die Tendenz des flüssigen Mediums, rasch stromabwärts zu strömen.

Grenzflächenaktive Verbindungen nach der Erfindung sind in den Ansprüchen 35 und 36 definiert. Sie umfassen Stoffe, die hydrophile Gruppen, beispielsweise -COOMe, - OSO₃Me, -SO₃Me, -NH₂, =NH, -NR₃⁺ und hydrophobe Alkylketten mit 10 bis 18 C-Atomen oder Alkylaryl-Reste enthalten. Bevorzugt gebraucht man nach Anspruch 36 Substanzen mit den Summenformeln C₂₀H₃₇NaO₇S oder C₁₈H₂₉NO₃S.

Um der erfindungsgemäßen Vorrichtung die nötige Steifheit bei gleichzeitig geringem Gewicht zu verleihen, ist die Verwendung von Polymeren angebracht. Dabei ist es besonders günstig, wenn das polymere Material nach Anspruch 37 ein Polyethylen, ein Polystyrol, ein Polyurethan, ein Polyvinylacetat, ein Polyester, beispielsweise Polyethylenterephthalat, ein Epoxyharz, ein Methacryl-Polymer, ein Polycarbonat, ein Polyvinylchlorid oder ein Copolymer der genannten Verbindungen ist. Man vermeidet übermäßigen Materialverbrauch, wenn das polymere Material in einer Erweiterung nach Anspruch 38 eine bevorzugt aus Polyester, Polycarbonat oder Polyvinylchlorid gefertigte Folie ist.

Ein wesentlicher Teil der Erfindung ist in Anspruch 39 formuliert. Dieser offenbart ein Verfahren zum Herstellen einer Vorrichtung zum Erfassen und zur Bestimmung der Konzentration wenigstens eines in einem flüssigen Medium gelösten Analyten. Erfindungsgemäß wird hierzu ein Elektrodenmaterial zu einer Paste verarbeitet und diese auf einen Träger derart aufgebracht, daß ein Mehrfach-Elektrodensatz aus wenigstens einer Arbeitselektrode und aus wenigstens einer Referenzelektrode sowie aus daran angeschlossenen Leiterbahnen und Kontakten gebildet wird. Dann wird der mit dem Mehrfach-Elektrodensatz versehene Träger bei erhöhter Temperatur getrocknet und anschließend werden auf ihn Liganden und/oder biologisch aktive spezifische Bindungsmoleküle und/oder katalytisch aktive Proteine aufgebracht. Dies geschieht im Bereich des Mehrfach-Elektrodensatzes oder in einem Bereich, der stromaufwärts des Mehrfach-Elektrodensatzes angeordnet ist. Auf den so erhaltenen Träger wird eine längliche Unterstützung, eine Abdeckung und ein Flüssigkeitsaufnahmeelement so befestigt, daß die Abdeckung und der Träger zueinander einen Abstand haben. Eine auf diese Weise hergestellte Meßvorrichtung kann von einer Lösung durchflossen werden und dabei gleichzeitig ein bestimmtes Lösungsvolumen im Bereich der Elektroden zurückhalten. Außerdem lassen sich nun Analyten, die für biologisch aktive spezifische Bindungsmoleküle oder Liganden spezifisch sind, in exakter Weise bestimmen. Sie werden gezielt aus der Meßlösung abgetrennt und auf dem Träger an oder nahe bei den Elektroden gebunden. Schließlich läßt sich durch die Verwendung von leitenden Pasten sicherstellen, daß Elektroden sowie Leiterbahnen und Kontakte in den verschiedensten Formen, Größen und Anordnungen hergestellt werden können.

Es ist wesentlich für die Erfindung, daß wenigstens eine Arbeitselektrode mit spezifischen Molekülen versehen wird. Dies geschieht nach Anspruch 40 dadurch, daß die Paste Liganden und/oder biologisch aktive spezifische Bindungsmoleküle und/oder katalytisch aktive Proteine enthält und mittels Siebdrucktechnik aufgetragen wird. Diese Vorgehensweise ist zeitsparend, denn eine nachträgliche Beschichtung von Arbeitselektroden entfällt. Sie ist jedoch nur praktikabel, wenn die Liganden bzw. die biologisch aktiven spezifischen Bindungsmoleküle sowie die katalytisch aktiven Proteine temperaturbeständig sind und beim Trocknungsprozeß nicht zerstört werden. Weil dies jedoch nicht für alle eingesetzten Moleküle gilt, verfährt man ergänzend nach Anspruch 41. Dieser bestimmt, daß wenigstens eine der getrockneten Elektroden mit Liganden und/oder biologisch aktiven spezifischen Bindungsmolekülen und/oder katalytisch aktiven Proteinen vorzugsweise unter Einsatz von Vernetzungsmolekülen oder Kupplungsreagenzien beschichtet wird. In gleicher Weise wird nach Anspruch 42 wenigstens eine der getrockneten Elektroden mit Liganden und/oder reaktionsunspezifischen Bindungsmolekülen vorzugsweise unter Einsatz von Vernetzungsmolekülen oder Kupplungsreagenzien beschichtet.

Die Meßprobe strömt schneller den Träger entlang, wenn man stromabwärts angeordnete Bereiche der Meßvorrichtung hydrophil gestaltet. Deshalb wird das Flüssigkeitsaufnahmeelement nach Anspruch 43 mit einer Lösung eines grenzflächenaktiven Stoffes behandelt und getrocknet, wobei dieser Stoff in einer bevorzugten Ausführungsform eine Verbindung der Summenformel C₂₀H₃₇NaO₇S oder C₁₈H₂₉NO₃S ist und in einer Konzentration von 0,001 bis 5 % w/v, vorzugsweise von 0,1 bis 3 % w/v eingesetzt wird.

Anspruch 44 offenbart ein Verfahren zum Erfassen sowie zur Bestimmung der Konzentration wenigstens eines in einem flüssigen Medium gelösten Analyten mit einer Vorrichtung, die aus wenigstens einer Arbeitselektrode und wenigstens einer Referenzelektrode besteht. Erfindungsgemäß bringt man eine definierbare Menge des flüssigen Mediums auf eine Probenauftragszone der Vorrichtung auf. Dann wartet man eine vorgebbare Zeit ab, um eine Wechselwirkung des/der Analyten mit Liganden und/oder biologisch aktiven spezifischen Bindungsmolekülen und/oder katalytisch aktiven Proteinen stattfinden zu lassen. Anschließend bringt man auf die Probenauftragszone eine Lösung auf, die wenigstens ein für ein katalytisch aktives Protein oder für mehrere katalytisch aktiven Proteine reaktionsspezifisches Substrat enthält und verbindet die Vorrichtung mit einem elektrischen Meßgerät. Nach einer Inkubationszeit liest man auf dem an die Vorrichtung angeschlossenen Meßgerät für jeden zu bestimmenden Analyten ein elektrisches Signal ab und ordnet ihm anhand einer Eichkurve eine entsprechende Konzentration des jeweiligen Analyten zu. Für eine Messung sind nur zwei Lösungen erforderlich; eine Analytbestimmung kann somit einfach und schnell durchgeführt werden. Weil Waschflüssigkeiten nicht benötigt werden, ist das Verfahren sehr anwendungsfreundlich.

Anspruch 45 belegt, wie schnell das erfindungsgemäße Verfahren ist und wie wenig flüssiges Medium benötigt wird. So beträgt dessen definierbare Menge 0,01 bis 20 µl, und vorzugsweise 1 bis 10 µl. Die vorgebbare Zeit liegt zwischen 5 und 600 Sekunden, vorzugsweise zwischen 10 und 120 Sekunden und in einer ganz bevorzugten Form bei 20 bis 60 Sekunden. Der geringe Flüssigkeitsverbrauch zeigt sich auch bei den Mengen an spezifischem Substrat gemäß Anspruch 46. Auf die Probenauftragszone müssen lediglich zwischen 0,1 µl und 500 µl, vorzugsweise zwischen 1 µl und 100 µl, und am bevorzugtesten Mengen von 10 µl bis 30 µl dieser Lösung aufpipettiert werden. Die sich daran anschließende Inkubationszeit liegt nach Anspruch 47 in einem Bereich von 2 bis 100 Sekunden, vorzugsweise von 5 bis 30 Sekunden und am bevorzugtesten bei 20 Sekunden und ist somit ebenfalls recht kurz.

Die Genauigkeit der Analytbestimmung läßt sich insbesondere bei Lösungen mit vielen elektroaktiven Teilchen durch die Verfahrensweise nach Anspruch 48 deutlich verbessern. Hierzu vergleicht man das abgelesene elektrische Signal mit einem weiteren elektrischen Signal, welches von einer zweiten, unspezifischen Arbeitselektrode stammt, wobei diese zweite Arbeitselektrode nur mit reaktionsunspezifischen Liganden oder Bindungsmolekülen versehen ist.

Die erfindungsgemäße Vorrichtung verwendet man in Einklang mit Anspruch 49 zum Erfassen sowie zur Bestimmung der Konzentration wenigstens eines Analyten aus Körperflüssigkeiten wie Vollblut, Plasma, Serum, Urin, Sekret, Liquor, aus Extrakten von Körpergeweben und aus Abstrichmaterial.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- Fig. 1: eine Draufsicht auf eine Meßvorrichtung bestehend aus einer Referenzelektrode und einer Arbeitselektrode,
- Fig. 2: eine Seitenansicht der Meßvorrichtung aus Fig. 1 entlang der Linie A-A,
- Fig. 3: eine Draufsicht auf eine Meßvorrichtung mit zwei Arbeitselektroden und
- Fig. 4: eine Draufsicht auf eine Meßvorrichtung mit drei Arbeitselektroden.

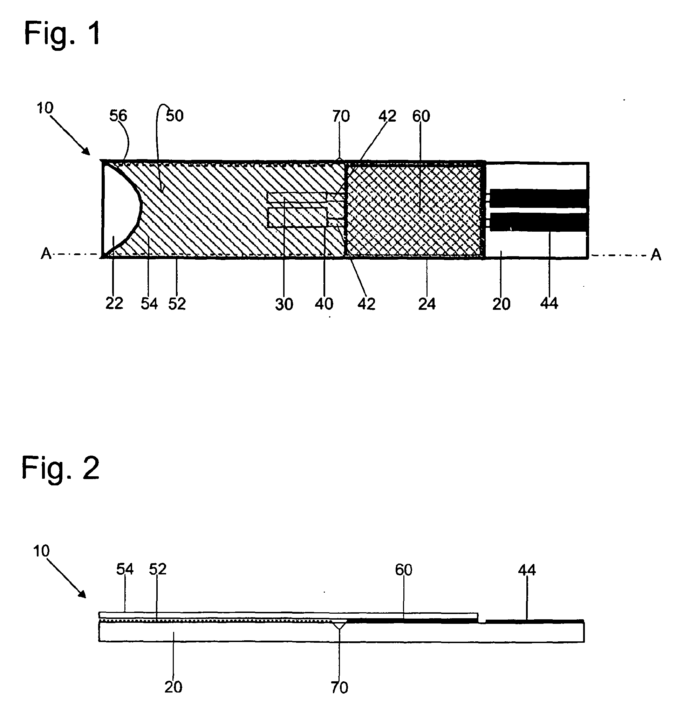

Die in Fig. 1 allgemein mit 10 bezeichnete Meßvorrichtung verwendet man zur qualitativen und quantitativen Bestimmung von gelösten Analyten. Sie besteht aus einem Träger **20,** auf dem in einer Grundausführung ein Elektrodensatz aus einer Arbeitselektrode **40** und einer Referenzelektrode **30** aufgebracht ist. Die Elektroden **30, 40** liegen in einem Reaktions- und Detektionsraum **50.** Dieser besteht aus einem Teil des Trägers **20,** einer länglichen Unterstützung **52** und einer Abdeckung **54.** Er ist an zwei Seiten offen. Die Abdeckung **54** besitzt an einer dieser offenen Seiten eine Aussparung **56,** die eine Probenauftragszone **22** auf dem Träger **20** freigibt. An der anderen Seite folgt auf die Abdeckung **54** ein Zwischenraum **70,** der seinerseits durch ein Flüssigkeitsaufnahmeelement **60** begrenzt wird.

Im Reaktions- und Detektionsraum **50** sind biologisch aktive spezifische Bindungsmoleküle vorhanden, die mit einem gesuchten Analyten wechselwirken können und teilweise wenigstens ein katalytisch aktives Protein tragen.

Zur Bestimmung der Konzentration des Analyten trägt man auf die Probenauftragszone **22** eine Meßprobe auf. Diese wandert durch Kapillarwirkung stromabwärts in den Reaktions- und Detektionsraum **50.** Der Analyt reagiert dort mit einem biologisch aktiven spezifischen Bindungsmolekül, welches ein katalytisch aktives Protein trägt. Es bildet sich ein Komplex, der sich weiter stromabwärts bis an die Elektroden **30, 40** bewegt, wo er mit einem immobilisierten, biologisch aktiven spezifischen Bindungsmolekül wechselwirkt. Auf diese Weise werden die gesuchten Analytmoleküle an oder auf wenigstens einer Elektrode **40** konzentriert.

Die übrige Meßprobe verteilt sich weiter stromabwärts in den Zwischenraum **70** und wird vom angrenzenden Flüssigkeitsaufnahmeelement **60** teilweise aufgesogen. In einem zweiten Schritt bringt man auf die Probenauftragszone **22** eine Lösung auf, die wenigstens ein reaktionsspezifisches Substrat für wenigstens ein katalytisch aktives Protein enthält. Diese dringt ebenfalls in den Reaktions- und Detektionsraum **50** ein und verdrängt ungebundene Bestandteile der Meßprobe in den Zwischenraum **70** und das Flüssigkeitsaufnahmeelement **60.** Das Substrat reagiert mit dem katalytisch aktiven Protein. Katalytisch aktive Proteine verbleiben nur im Reaktions- und Detektionsraum **50,** wenn das an sie gebundene biologisch aktive spezifische Bindungsmolekül mit dem Analyten bindend wechselwirkt und dieser wiederum durch das immobilisierte biologisch aktive spezifische Bindungsmolekül ortsfixiert ist. Deshalb ist die Menge an umgesetztem reaktionsspezifischem Substrat proportional zur Konzentration an katalytisch aktivem Protein im Reaktions- und Detektionsraum **50** und somit ein Maß für die Menge an gesuchtem Analyt in der Meßprobe.

Das katalytisch aktive Protein überführt das zugehörige reaktionsspezifische Substrat in eine oxidierte oder reduzierte, jedenfalls elektroaktive Substratform. Diese wechselwirkt mit der Arbeitselektrode **40,** wodurch ein elektrisches Signal in Form einer Strom- und/oder Spannungsänderung erzeugt wird. Diese ist proportional zur Menge an erzeugter elektroaktiver Substratform und somit ein quantitatives Maß für die Menge an Analyt in einer Meßlösung. Um reproduzierbare Meßwerte zu erhalten, ist es erforderlich, daß im Reaktions- und Detektionsraum **50** stets ein konstantes Meßvolumen vorhanden ist. Dies wird dadurch sichergestellt oder zumindest unterstützt, daß zwischen Flüssigkeitsaufnahmeelement **60** und Reaktions- und Detektionsraum 50 der Zwischenraum **70** vorgesehen ist.

Der Träger **20** der Meßvorrichtung **10** besteht aus einer flexiblen, vorzugsweise selbsttragenden und nichtleitenden Kunststofflage. Diese ist einstückig und aus einem Polymer oder einem Copolymer aus zweien oder mehreren der weiter unten genannten polymeren Materialien aufgebaut. In einer besonderen Erweiterung besitzt sie eine mikroskopische Oberflächenstruktur, durch die neben Flüssigkeiten und kleinen Antigenen auch gelöste biologische Makromoleküle wie beispielsweise Enzyme, globuläre Proteine, Nukleinsäuren, Infektionserreger und Antikörper leicht hindurchdiffundieren können. Der vorzugsweise in Folienform und rechteckig ausgeführte Träger **20** besitzt zwei Längskanten **24** und ist so dick, daß er ohne eine zusätzliche Stützschicht in eine Meßvorrichtung **10** eingebaut werden kann, beispielsweise zwischen 0,1 mm und 5 mm. Er kann jedoch entlang seiner Längsausdehnung auch unregelmäßig ausgeführt sein und breiter dimensionierte Bereiche aufweisen. Außerdem kann die Kunststofflage sich in einer Richtung verjüngen und/oder abgerundet sein.

Um gezielt biologisch aktive spezifische Bindungsmoleküle an bestimmten Stellen, beispielsweise im Reaktions- und Detektionsraum **50,** mit einer reaktionsträgen Kunststofflage verbinden zu können, bringt man zuvor auf den Träger **20** eine Beschichtung auf. Hierzu behandelt man ihn sozusagen als Grundierung mit einer Lösung, deren Bestandteile sich elektrostatisch bzw. mittels van der Waalsscher Kräfte an dessen Oberfläche binden. Polyaminocarbonsäuren verändern beispielsweise eine Polyethylenfolie chemisch derart, daß sie biologisch aktive spezifische Bindungsmoleküle kovalent binden kann.

Zur Oberflächenaktivierung verwendet man neben den genannten Säuren aber auch Proteinlösungen tierischer oder menschlicher Albumine, Globuline oder Glykoproteine, sowie Zubereitungen aus bakteriellen Proteinen. Beispiele für Albumine sind Lactalbumin und Humanserum-Albumin, sowie Serumalbumine vom Rind, Kaninchen, Schaf, Pferd, vom Schwein und von der Ziege. Als Glykoprotein setzt man beispielsweise Ovomucoid ein und die verwendeten bakteriellen Proteine isoliert man vorzugsweise aus Streptomyces avidinii. Neben Proteinen kommen jedoch auch Proteide zum Einsatz, sprich Proteine, die mit weiteren Gruppen beispielsweise mit Kohlenhydrat-Nukleinsäure- oder Lipidresten versehen sind.

Darüber hinaus werden Polyaminosäuren oder Polypeptide verwendet, wobei letztere bevorzugt Abbauprodukte von Proteinen des Stütz- und Bindegewebes sind, wie etwa das aus Gelatine gewonnenes Gelafusal^{®}.

Eine besonders gute Haftgrundlage wird erreicht, wenn die verwendeten Proteine, Proteidie, Polyaminosäuren oder Polypeptide infolge Denaturierung in einer "random coil" Konformation auf dem Träger **20** angeordnet sind. Hierzu erhitzt man eine Lösung von ihnen vor dem Auftragen auf 50 bis 80 °C oder setzt ihr chaotrope Agenzien wie Harnstoff oder Guanidinsalze zu. Gleiches wird durch Ändern des pH-Wertes, durch Ultraschall, Versetzen mit organischen Solvenzien oder durch Behandlung mit ionisierender Strahlung erreicht.

Die am stromaufwärtigen Ende des Trägers **20** befindliche Probenauftragszone **22** besteht in einer Grundausführung nur aus einem offenliegenden halbkreisförmigen Trägerteilbereich. Sie ist so ausgeführt, daß eine Meßprobe mühelos aufpipettiert oder aufgetropft werden kann. In einer nicht gezeichneten Erweiterung setzt sie sich jedoch aus mehreren, voneinander abgetrennten Bereichen zusammen, deren Aussehen durch die Form der Aussparung **56** beziehungsweise durch nicht gezeichnete zusätzliche Ausstanzungen in der Abdeckung **54** vorgegeben ist.

Damit die Meßprobe möglichst schnell in den Reaktions- und Detektionsraum **50** strömt, behandelt man die Probenauftragszone **22** mit grenzflächenaktiven Verbindungen wie beispielsweise Detergenzien. Trägt man diese so auf, daß ihr hydrophiler Charakter in stromabwärtiger Richtung ansteigt, erreicht man eine besonders hohe Einströmgeschwindigkeit. In einer Weiterführung liegt auf der Probenauftragszone **22** ein nicht dargestelltes maschenartiges Material, beispielsweise ein mit Detergenzien behandeltes Nylongewebe auf und zerteilt einen zu untersuchenden Meßtropfen. Werden mehrere solcher Netzgewebe übereinander gelegt, fördert dies zusätzlich die gleichmäßige Verteilung einer Meßprobe auf der Probenauftragszone **22** und somit ein schnelles Einströmen. Dieser Effekt ist besonders ausgeprägt, wenn die einzelnen Gewebe unterschiedlich große Maschenweiten haben und beginnend mit der kleinsten Maschenweite übereinander geschichtet werden.

Auf dem beschichteten Träger **20** sind im stromabwärtigen Bereich Mittel zur Ableitung elektrischer Signale angeordnet, die in einer nicht gezeichneten Ausführung gegeneinander isoliert sind. Sie bestehen aus Elektroden **30, 40,** wobei jede von ihnen über eine eigene Leiterbahn **42** mit einem am stromabwärtigen Ende des Trägers **20** angeordneten Kontakt **44** verbunden ist.

Jede Elektrode **30, 40** ist gegenüber den schlank und langgezogenen Leiterbahnen **42** breiter ausgebildet, so daß eine möglichst große Oberfläche für Wechselwirkungen mit gelösten Substanzen einer Meßlösung besteht. Nach Fig. 1 besitzen alle Elektroden **30, 40** eine rechteckige Form. In einer Erweiterung können sie jedoch auch quadratisch, kreisrund, als stromaufwärts geschlossener bzw. geöffneter Halbkreis oder als Vieleck ausgebildet sein. Allgemein beansprucht jede Elektrode **30, 40** einen Flächenbereich von 0,2 bis 20 mm², vorzugsweise von 1 bis 5 mm², wobei die Arbeitselektrode **40** in der Regel mehr Fläche als die Referenzelektrode **30** einnimmt. In Fig. 1 erkennt man, daß die Arbeitselektrode **40** etwa dreimal mehr Platz benötigt.

Alle mit Strom und/oder Spannung beaufschlagbaren Teile **30, 40, 42, 44** bestehen aus Kohlenstoff - vorzugsweise Graphit - bzw. aus Silber, Platin, Gold, Palladium, Titan Nickel oder Kupfer. Sie werden, sofern die Ausgangsstoffe als Pulver oder Paste vorliegen, nach Möglichkeit in einem Schritt mittels Siebdruck auf den Träger **20** aufgebracht. Bei einer anderen Bauform wird ein aus einer Elektrode **30, 40** mit zugehöriger Leiterbahn **42** samt Kontakt **44** gefertigtes Bauteil aus metallischem Flachmaterial hergestellt und aufgeklebt.

Die Referenzelektrode **30** ist nicht mit teilchenselektiven Gruppen oder Verbindungen versehen. Sie hat den Aufbau einer Ag/AgCl-Elektrode. Für bestimmte Anwendungen besteht sie jedoch aus wenigstens einer Kohlenstoffmodifikation und/oder aus den vorab genannten Elementen, die sowohl einzeln als auch vergesellschaftet verwendet werden. Alle Leiterbahnen **42** und Kontakte **44** werden mit einer kohlenstoffhaltigen Graphitpaste hergestellt (z.B. Ercon E0455-125 graphite ink).

Die Arbeitselektrode **40** wird vorzugsweise aus Graphitpaste gefertigt und besitzt an ihrer Oberfläche immobilisierte Liganden oder immobilisierte Liganden, an die biologisch aktive spezifische Bindungsmoleküle gebunden sind. In einer Ausgestaltung trägt sie zusätzlich immobilisierte katalytisch aktive Proteine, die entweder unmittelbar auf ihrer Oberfläche vorhanden und/oder an Liganden bzw. an biologisch aktive spezifische Bindungsmoleküle gebunden sind. Die Komponenten Arbeitselektrode **40,** Ligand, biologisch aktives spezifisches Bindungsmolekül und katalytisch aktives Protein sind kovalent, elektrostatisch, mittels Wasserstoffbrücken oder durch van der Waalssche Kräfte miteinander verbunden.

In einer weitergebildeten Ausführungsform sind zwei katalytisch aktive Proteine mit unterschiedlicher Spezifizität permanent an die Oberfläche der Arbeitselektrode **40** gebunden. In einer Ausgestaltung dieser Ausführungsform ist das erste Protein mit einem Liganden oder mit einem biologisch aktiven, spezifischen Bindungsmolekül verbunden, während das zweite unmittelbar auf der Oberfläche der Arbeitselektrode **40** immobilisiert ist. Diese beiden Zwei-Protein-Anordnungen sind günstig, wenn der zu bestimmende Analyt ein reaktionsspezifisches Substrat ist. Jedes die Arbeitselektrode **40** erreichende Substrat wird vom ersten katalytisch aktiven Protein zu einer Zwischenstufe umgesetzt, die mit dem zweiten katalytisch aktiven Protein reagiert (Reaktionskaskade). Hierbei entsteht die elektroaktive Substratform, deren Menge durch die Größe des an der Arbeitselektrode **40** erzeugten elektrischen Signals festgestellt wird und die Mengen des in der Meßprobe befindliche Analyten wiedergibt.

Bei einer noch anderen Ausgestaltung trägt die Arbeitselektrode **40** einen Liganden oder einen Liganden mit biologisch aktivem spezifischen Bindungsmolekül und außerdem ausschließlich das zweite katalytisch aktive Protein. Dieses ist unmittelbar auf der Arbeitselektrode **40** oder aber an den Liganden bzw. das biologisch aktive spezifische Bindungsmolekül gebunden und sorgt wiederum für die Bildung der elektroaktiven Substratform in einer Reaktionskaskade, wobei das erste katalytisch aktive Protein sich auf dem Träger **20** befindet.

Zur Bestimmung eines einzigen Analyten, also zur Schaffung eines Ein-Analyt-Sensors gemäß Fig. 1 ist wenigstens eine Arbeitselektrode **40** und wenigstens eine Referenzelektrode **30** erforderlich.

Teilchenselektive Gruppen oder Verbindungen, sprich Liganden, biologisch aktive spezifische Bindungsmoleküle und/oder katalytisch aktive Proteine, können einzeln oder miteinander verknüpft nicht nur an der Arbeitselektrode **40** gebunden vorliegen, sondern auch in verschiedenen Bereichen auf dem Träger **20** angeordnet sein. Diese Anordnung beruht auf einer kovalenten, einer elektrostatischen, einer mittels Wasserstoffbrücken erzeugten oder aber auf einer durch van der Waalssche Kräfte hervorgerufenen Wechselwirkung mit der Kunststofflage. Sie sorgt dafür, daß teilchenselektive Gruppen oder Verbindungen auf dem Träger **20** aufgetrocknet und daher in feuchtem Zustand beweglich und/oder auf ihm permanent fixiert und daher im feuchten Zustand nicht beweglich sind.

Liganden haben zwei Funktionen. Entweder binden sie unmittelbar einen bestimmten Analyten an sich oder sie dienen als Brückenglied zwischen Trägeroberfläche bzw. Arbeitselektrodenoberfläche einerseits und biologisch aktivem spezifischem Bindungsmolekül andererseits.

In einer Ausgestaltung der Erfindung wird die Meßvorrichtung **10** mit einer Lösung behandelt, die Liganden in Konzentrationen von 0,0007 bis 0,7 mol/l und vorzugsweise von 0,035 bis 0,35 mol/l enthält. Dabei handelt es sich um eine Verbindung aus der Familie der Aminocarbonsäuren, insbesondere der Diaminocarbonsäuren.

Gleichsam geeignet sind höhermolekulare, homodet (= ausschließlich über Peptidbindungen) oder heterodet (= nicht ausschließlich über Peptidbindungen) verknüpfte Polyaminocarbonsäuren. Sie haben die allgemeine Strukturformel R-CH(NH₂)-(CH₂)ₙ-COOH, wobei R ein Proton sowie eine Amino-, Imino-, Hydroxy-, Thiol-, Hydroxyalkyl-, Aminoalkyl-, oder Carboxyalkyl-Gruppe umfaßt und n einen Wert von 0 bis 6, vorzugsweise 0 annimmt.

Quellen für höhermolekulare peptidartig verknüpfte Verbindungen sind depolymerisierte Hydrolyseprodukte fibrillärer Skleroproteine des tierischen Bindegewebes. Ein Beispiel hierfür ist das aus Kollagen durch oxidativ-thermische Abbauvorgänge gewonnene Gelafusal^{®}. Sollen Proteine als Liganden eingesetzt werden, gewinnt man diese u.a. aus dem Bakterium Streptomyces avidinii.

Weiterhin lassen sich Ribonucleinsäuren oder Desoxyribonucleinsäuren und verschiedene Boronsäuren wie beispielsweise Phenylboronsäure als Ligand verwenden.

Auch Verbindungen auf Kohlenhydratbasis, wie beispielsweise Concanavalin A, verschiedene Lektine oder Agglutinine wie Hämagglutinin sowie Streptavidin sind als Liganden einsetzbar.

Eine Ausführungsform der Meßvorrichtung sieht auf dem gesamten Träger **20** polymere Aminocarbonsäuren als immobilisierte Liganden vor. In unmittelbarer Nähe zur einer Arbeitselektrode **40** oder auf ihr sind biologisch aktive spezifische Bindungsmoleküle kovalent an die Liganden gebunden und deshalb unbeweglich. Im Bereich oder jenseits der Probenauftragszone **22,** also stromaufwärts der Elektroden **30, 40** befinden sich im feuchten Zustand bewegliche biologisch aktive spezifische Bindungsmoleküle, die ein katalytisch aktives Protein tragen. Der gesuchte Analyt aus der Meßprobe bindet an eines der beweglichen Bindungsmoleküle und ein dadurch gebildeter beweglicher Komplex diffundiert infolge Kapillarwirkung in den Reaktions- und Detektionsraum **50,** um dort an eines der kovalent gebundenen Bindungsmoleküle zu binden.

In einer anderen Ausführung ist der Träger **20** frei von Liganden oder enthält sie nur in ausgezeichneten Bereichen. Der bewegliche Komplex besteht aus einem biologisch aktiven spezifischen Bindungsmolekül und einem katalytisch aktiven Protein sowie gegebenenfalls aus einem beweglichen Liganden. Nach Aufnahme des Analyten wandert dieser Komplex infolge Kapillarwirkung auf dem Träger **20** in den Reaktions- und Detektionsraum **50** und bindet dort an einen immobilisierten Liganden oder an ein immobilisiertes biologisch aktives spezifisches Bindungsmolekül. Dieses kann ebenfalls mit einem Liganden verknüpft sein.

Zum Erhalt der biologischen und immunologischen Aktivität der biologisch aktiven spezifischen Bindungsmoleküle und der katalytisch aktiven Proteine, bettet man diese auf dem Träger **20** in Polyhydroxyverbindungen oder polymere Aminocarbonsäuren oder besser in ein Gemisch daraus ein. Hierdurch wird eine für kommerzielle Produkte geforderte Langzeitstabilität erreicht.

Biologisch aktive spezifische Bindungsmoleküle sind in der Lage, einen Analyten selektiv an sich zu binden. Je nach Verwendung sind sie mit einem katalytisch aktiven Protein und/oder mit einem Liganden verknüpft. Man kann sie auf dem Träger **20** permanent fixieren oder aber so aufbringen, daß sie im trockenen Zustand unbeweglich sind und sich bei Befeuchtung entlang des Trägers **20** fortbewegen. Sie lassen sich auch unlösbar an einen Liganden binden, der bereits auf dem Träger **20** immobilisiert ist.

Oft verwendet man zwei biologisch aktive spezifische Bindungsmoleküle, von denen eines beweglich stromaufwärts auf dem Träger **20** vorliegt und mit einem katalytisch aktiven Protein kovalent zu einem Konjugat verknüpft ist, während das zweite permanent haftend stromabwärts in der Nähe der Arbeitselektrode **40** oder auf ihr immobilisiert ist. So ist gewährleistet, daß Analyt und Konjugat in entsprechend proportionaler Menge in die Nähe der Arbeitselektrode **40** oder an sie heran diffundieren und dort dauerhaft festgehalten werden. Es bildet sich ein Sandwich aus Konjugat, Analyt und immobilisiertem biologisch aktivem spezifischem Bindungsmolekül.

Unter die Gruppe der biologisch aktiven spezifischen Bindungsmoleküle lassen sich alle Verbindungen zusammenfassen, die einen Analyten oder ein Antigen bzw. deren haptene Komponente selektiv binden ohne sie jedoch chemisch umzusetzen. Hierunter fallen insbesondere monoklonale und polyklonale Antikörper und Antigene.

Als katalytisch aktive Proteine setzt man meist Enzyme oder Rekombinanten aus Antikörpern und Enzymen ein. So werden bevorzugt Oxidoreduktasen, insbesondere Glucose-Dehydrogenase, Glucoseoxidase und Peroxidase verwendet, wobei jedoch auch andere Enzyme sich genauso eignen.

Um die genannten Verbindungen auf dem Träger **20** in einer nativen Konformation zu erhalten, kann man gelöste Polyhydroxyverbindungen, beispielsweise Mono- Oligo- oder Polysaccharide zugeben. Die native Struktur läßt sich auch mit einer Lösung aus Proteinen und Hydroxyverbindungen konservieren. Diese verwendet man jedoch auch zum Aktivieren des Trägers **20.**

In Fig. 2 erkennt man, daß der Träger **20** mit seinen Mitteln zur Ableitung elektrischer Signale vor äußeren Einflüssen geschützt ist. Hierzu wird auf dessen Längskanten **24** die längliche Unterstützung **52** in Form zweier gegenüberliegender Streifen aufgebracht. Diese bestehen entweder aus jeweils einer Schicht Schmelzkleber oder aus einem polymeren Material. Sie erstrecken sich nur über einen Teil der beiden Trägerlängskanten **24** bis an den Zwischenraum **70.** In einer nicht gezeichneten Variante können sie jedoch, entlang der gesamten Trägerlänge **24** verlaufen und sich sogar noch teilweise entlang der Schmalseiten des Trägers **20** ausdehnen. Dies macht zum einen die Probenauftragszone **22** kleiner und stützt und schützt zum anderen die Kontakte **44.** Will man in einer Weiterführung dieser Variante eine gute Belüftung der Meßvorrichtung **10** erreichen, so ist die Unterstützung **52** im Bereich des Zwischenraums **70** unterbrochen.

Auf der länglichen Unterstützung **52** liegt die Abdeckung **54** permanent haftend auf, wodurch stromauf des als Luftspalt wirkenden Zwischenraums **70** der Reaktions- und Detektionsraum **50** entsteht. Die Abdeckung **54** ist in der Ausführung von Fig. 2 so ausgelegt, daß sie den Zwischenraum **70** überragt und sich auf dem Flüssigkeitsaufnahmeelement **60** abstützt. In zwei nicht gezeichneten Varianten kann jedoch nur der Reaktions- und Detektionsraum **50** oder aber der Reaktions- und Detektionsraum **50** mitsamt dem Zwischenraum **70** abgedeckt werden. Schließlich kann die als Haube wirkende Abdeckung **54** auch ausschließlich auf der länglichen Unterstützung **52** aufliegen oder sogar mit ihr einstückig sein.

Gewöhnlich besteht die vorzugsweise einstückige Abdeckung **54** aus einem der weiter unten genannten polymeren Materialien, das aus konstruktiven Gründen nach Möglichkeit mit dem des Trägers **20** identisch ist. Sie besitzt wenigstens eine schlitz- oder halbkreisförmige Öffnung **56** durch die wenigstens eine Meßprobe oder wenigstens eine reaktionsspezifisches Substrat auf den Träger **20** aufgebracht werden kann. Die Form der Öffnung(en) **56** kann beliebig gewählt werden und hängt von der Menge und Beschaffenheit aufzubringender Lösungen ab.

Der Reaktions- und Detektionsraum **50** befindet sich zwischen der Probenauftragszone **22** und dem Zwischenraum **70.** Er hat als starres Gebilde definierte Abmessungen und nimmt ein konstantes Flüssigkeitsvolumen in sich auf. Weil er in stromabwärtiger und stromaufwärtiger Richtung offen ist, kann die Meßprobe durch ihn hindurchfließen. In seinem stromabwärtigen Teil sind die Referenzelektrode **30** und wenigstens eine Arbeitselektrode **40** sowie gegebenenfalls Teile der Leiterbahnen **42** angeordnet. Werden große Meßprobenvolumina eingesetzt, ist der Reaktions- und Detektionsraum **50** größer gestaltet. Dies erreicht man dadurch, daß die Streifen der länglichen Unterstützung **52** dicker dimensioniert sind.

Die Meßprobe bzw. die das reaktionsspezifische Substrat enthaltende Lösung dringt besonders schnell und gleichförmig in den Reaktions- und Detektionsraum **50** ein, wenn man dessen durch den Träger **20,** die längliche Unterstützung **52** und die Abdeckung **54** gebildeten Innenflächen mit grenzflächenaktiven Stoffen wie beispielsweise Detergenzien behandelt. In einer vorteilhaften Weiterbildung dieses Merkmals befinden sich im Bereich der Probenauftragszone **22** an den Innenflächen hydrophobe grenzflächenaktive Stoffe. Deren Menge nimmt kontinuierlich zugunsten einer hydrophilen Detergenzienschicht ab, je weiter man sich stromab bewegt. Im Bereich der Elektroden **30, 40** befinden sich schließlich nur noch hydrophile Detergenzien auf den innenseitigen Flächen. Eine derartige Anordnung grenzflächenaktiver Stoffe im Reaktions- und Detektionsraums **50** bewirkt, daß wässrige Proben schnell in die Nähe der stromabwärts gelegenen Elektroden **30, 40** gelangen.

Der Zwischenraum **70** grenzt stromaufwärts an den Reaktions- und Detektionsraum **50** und stromabwärts an das Flüssigkeitsaufnahmeelement **60** an. Wie in Fig. 1 zu sehen, befindet er sich auf dem Träger **20** stromabwärts der Elektroden **30, 40.** Er ist ein erfindungswesentliches Merkmal, denn er trägt dazu bei bzw. sorgt dafür, daß immer nur ein genau definiertes Meßprobenvolumen im Reaktions- und Detektionsraum 50 vorhanden ist. Als Luftspalt beeinflußt er zudem das Fließverhalten einer Meßprobe auf dem Träger **20.** Der Zwischenraum **70** ist in seiner Länge variabel und auf die Menge an Probenflüssigkeit abgestimmt. Größere Probemengen erfordern einen weiteren Abstand zwischen Reaktions- und Detektionsraum **50** und Flüssigkeitsaufnahmeelement **60,** demnach also einen größeren Zwischenraum **70,** kleine Meßproben hingegen einen kleineren.

In einer nicht gezeichneten Ausgestaltung ist der Zwischenraum **70** lateral durch die längliche Unterstützung **52** begrenzt, Darin eingebrachte Schlitze, vorzugsweise zwischen Reaktions- und Detektionsraum **50** und Flüssigkeitsaufnahmeelement **60** gewährleisten eine fein regulierte Belüftung und verändern somit das Fließverhalten der Meßprobe.

Soll deren Fließeigenschaft zusätzlich beeinflußt werden, läßt sich die Trägeroberfläche im Bereich des Zwischenraums **70** mit ausgewählten grenzflächenaktiven Stoffen beschichten. Man wählt hierzu bevorzugt hydrophobe Verbindungen, so daß der mit hydrophilen Stoffen versehene Bereich des Reaktions- und Detektionsraumes **50** stromaufwärts durch die hydrophobe Probenauftragszone **22** und stromabwärts durch den hydrophoben Zwischenraum **70** eingerahmt ist.

Das Flüssigkeitsaufnahmeelement **60** schließt sich an den Zwischenraum **70** in stromabwärtiger Richtung an und besteht aus einem saugfähigen und porösen Material. Es nimmt die flüssige Meßprobe auf, die bereits den Reaktions- und Detektionsraum **50** und den Zwischenraum **70** durchströmt hat. In Fig. 2 bildet es mit der Abdeckung **54** stromabwärts eine bündig abschließende Kante, so daß die stromabwärts hierzu angeordneten Kontakte **44** frei liegen.

Für das Aufnahmeelement **60** eignen sich alle Materialien, die in der Lage sind, schnell Flüssigkeit zu adsorbieren. Beispiele hierfür sind fibrilläre Vliese oder Gewebe wie Papier, Zellstoff, Glasfasern, Cellulosematerialien oder poröse Kunststoffe wie Polypropylen, Polyethylen, Polyvinylidenfluorid, Ethylenvinylacetat, Acrylnitril und Polytetrafluorethylen. Um eine ausschließlich unidirektionale, sprich eine stromabwärts gerichtete Saugwirkung von fibrillären Materialien wie beispielsweise Papier zu erzielen, verlaufen die Fasern in einer weitergeführten Ausführungsform des Flüssigkeitsaufnahmeelements **60** parallel zur Fließrichtung der Meßprobe.

Eine besonders gute Flüssigkeitsaufnahme wird auch dadurch erzielt, daß man ein mit grenzflächenaktiven Stoffen vorbehandeltes Flüssigkeitsaufnahmeelement **60** verwendet. Dieses wird hierzu vor dem Aufbringen auf den Träger **20** beispielsweise mit gelösten Detergenzien benetzt oder getränkt. Es handelt sich dabei um hydrophile Verbindungen, sofern die Meßlösung hydrophil ist und hydrophobe Stoffe bei entsprechend hydrophober Meßprobe.

Die für die verschiedenen Bestandteile der Meßvorrichtung **10** verwendeten grenzflächenaktiven Stoffe bestehen aus wenigstens einer hydrophilen Gruppe und aus wenigstens einem hydrophoben Molekülteil. Bei letzterem Teil handelt es sich um Alkylketten mit 10 bis 18 Kohlenstoffatomen. Der hydrophile Charakter der grenzflächenaktiven Stoffe entsteht, weil die genannten Alkylgerüste mit wenigstens einer der polaren Gruppen -COOMe, -OSO₃Me, -SO₃Me, -NH₂, =NH, -N⁺(R)₃ versehen sind. Besonders gute Benetzungsergebnisse werden erzielt, wenn die auch als Detergenz bezeichnete grenzflächenaktive Substanz die Summenformel C₂₀H₃₇NaO₇S oder alternativ die Formel C₁₈H₂₉NO₃S besitzt.

Für den Träger **20,** die längliche Unterstützung **52** und die Abdeckung **54,** also für all jene Teile, die einen gehäuseartigen Anteil der Meßvorrichtung **10** bilden, verwendet man polymere Materialien. Diese stützen die Vorrichtung **10** und machen sie bis zu einem gewissen Grad biegesteif. Es handelt sich hierbei um Polymere aus der Gruppe der Polyethylene, Polystyrole, Polyurethane, Polyvinylacetate und der Polyester beispielsweise der Polyethylenterephthalate. Man verwendet auch Polymere aus der Gruppe der Epoxyharze, der Methacryl-Polymere, der Polycarbonate und der chlorierten Polyvinylverbindungen (beispielsweise Polyvinylchlorid). Auch Copolymere aus zweien oder mehreren der genannten Verbindungen kommen zum Einsatz. Zudem setzt man beispielsweise als Trägermaterial oder auf dem Träger **20** Nitrozellulose- und/oder Zelluloselagen ein, beispielsweise um mehrere Regionen mit unterschiedlichen Fließeigenschaften in der Meßvorrichtung **10** zu schaffen.

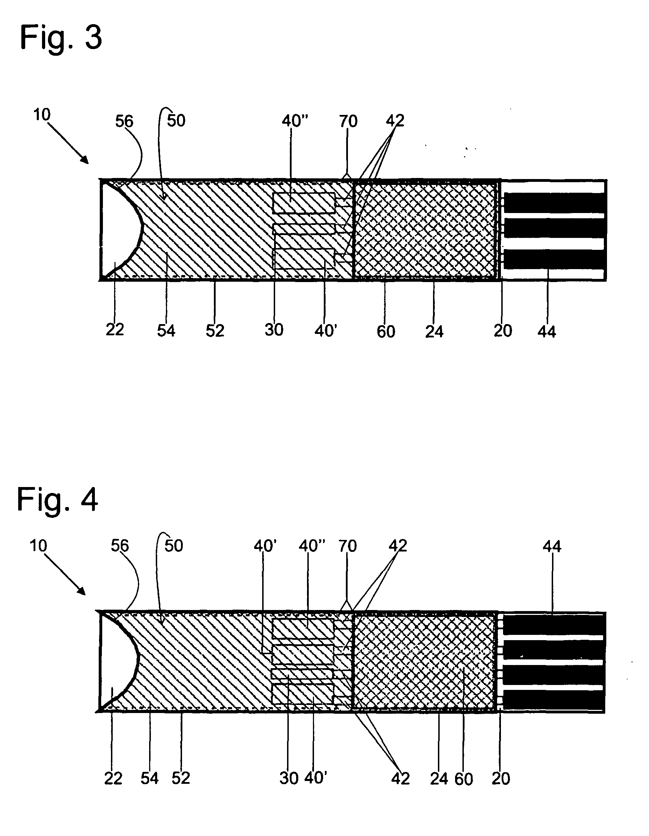

Eine weitere Ausführung der erfindungsgemäßen Meßvorrichtung **10** besteht aus einem als Folie ausgeführten Träger **20,** auf den drei Elektroden **30, 40, 40** aufgedruckt sind. Die gewöhnlich trägermittig angeordnete Referenzelektrode **30** ist von zwei Arbeitselektroden **40, 40** umgeben. Jede von ihnen **40, 40** ist mit unterschiedlichen Liganden und/oder biologisch aktiven spezifischen Bindungsmolekülen versehen, die ihrerseits wiederum an katalytisch aktive Proteine gebunden sein können. Diese Meßvorrichtung **10** läßt sich in zwei Bauformen unterteilen, und zwar in eine nicht gezeigte mit zwei spezifischen Arbeitselektroden **40', 40'** und in die in Fig. 3 abgebildete mit einer spezifischen und einer unspezifischen Arbeitselektrode **40', 40".**

Bei der ersten Bauform besitzt jede Arbeitselektrode **40', 40'** Liganden und/oder biologisch aktive spezifische Bindungsmoleküle, die jeweils selektiv einen Analyten aus der Meßprobe binden und detektieren können. Demnach weist man hiermit unabhängig voneinander zwei unterschiedliche Analyten innerhalb einer zu vermessenden Probe nebeneinander nach.

Die unspezifische Arbeitselektrode **40"** der zweiten Bauform ist mit unspezifischen biologisch aktiven Bindungsmolekülen vorzugsweise proteinogener Natur, beispielsweise mit Spezies Immunglobulinen versehen. Sie bindet verschiedenste Bestandteile der Meßprobe, die zu verfälschten Signalen an der spezifischen Arbeitselektrode **40'** führen können. Eine solche Anordnung ist dazu geeignet, ein durch unspezifische Bindung elektroaktiver Bestandteile hervorgerufenes Signal zu detektieren bzw. herauszufiltern und so die Sensitivität des Tests zu erhöhen.

Eine weitere Ausgestaltung der erfindungsgemäßen Meßvorrichtung **10** besitzt drei Arbeitselektroden **40, 40, 40.** Diese können für einen Analyten spezifisch und/oder unspezifisch sein. In der Regel sieht man gemäß Fig. 4 eine unspezifische **40"** und zwei spezifische Arbeitselektroden **40', 40'** vor. Deren Anordnung auf dem Träger **20** ist beliebig, wobei man jedoch darauf achtet, die Referenzelektrode **30** zentral zu plazieren.

Zur Herstellung der erfindungsgemäßen Meßvorrichtung **10** geht man von einem Träger **20** aus, der aus einem der genannten polymeren Materialien besteht. Dieser wird für bestimmte Anwendungen beschichtet, um eventuell vorhandene aktive Gruppen zu inaktivieren oder seine Oberfläche für die Bindung weiterer Moleküle vorzubereiten. In der Regel bringt man hierzu eine Grundierungslösung, welche die für die Beschichtung erforderlichen Bestandteile enthält, auf den Träger **20** auf und trocknet ihn anschließend.

Danach werden die Mittel zur Ableitung elektrischer Signale aufgebracht, sprich Elektroden **30, 40** Leiterbahnen **42** und Kontakte **44.** Hierzu legt man eine aus diesen Bestandteilen bestehende Anordnung auf der Trägeroberfläche ab oder bedruckt letztere nach dem Siebdruckverfahren mit den oben genannten elektrisch leitenden Pasten. Diese übertragen ein chemoelektrisches Signal besonders gut, wenn ihnen ein Mediator wie beispielsweise Ferrocen-Dicarbonsäure beigemischt ist. Darüber hinaus können sie bereits mit Liganden, biologisch aktiven spezifischen oder unspezifischen Bindungsmolekülen und/oder katalytisch aktiven Proteinen versetzt sein. Ein derartiges Vorgehen ist jedoch nur möglich, wenn die genannten teilchenselektiven Verbindungen ybei einer sich anschließenden Trocknung der Pasten bei erhöhter Temperatur beständig sind.

Sollten Liganden, biologisch aktive Bindungsmoleküle oder katalytisch aktive Proteine dem Trocknungsprozeß nicht oder nur zum Teil stand halten, müssen sie nachträglich als Schicht auf die Arbeitselektrode(n) **40** aufgebracht werden. Um sie kovalent auf einer Elektrodenoberfläche zu binden, verwendet man verschiedene Kupplungsreagenzien. Beispiele hierfür sind Glutaraldehyd, Säurechloride, Anhydride, Aktivester, Azide, Diazoverbindungen, Succinimide und Carbodiimide (Wong, S.S., Chemistry of Protein Conjugation and Cross-Linking. Florida: CRC Press, 1991). Bei diesen Substanzen kann es sich um homobifunktionelle oder heterobifunktionelle Verbindungen (cross-linker) handeln. Bifunktionell bedeutet, daß die Verbindungen zwei über eine Kohlenwasserstoffkette verbundene reaktive Gruppen besitzen: Homo besagt, daß diese Gruppen gleich und hetero, daß sie unterschiedlich sind.

Kupplungsreagenzien verwendet man auch, um Liganden und biologisch aktive Bindungsmoleküle und/oder katalytisch aktive Proteine, mit der beschichteten oder unbeschichteten Trägeroberfläche zu verbinden. Außerdem benötigt man sie, um die genannten Moleküle in allen denkbaren Kombinationen untereinander chemisch zu verknüpfen.

In einer Weiterführung der Erfindung trägt wenigstens eine Arbeitselektrode **40** keine teilchenselektiven Verbindungen. Die Liganden und/oder biologisch aktiven Bindungsmoleküle und/oder katalytisch aktiven Proteine sind stattdessen auf dem Träger **20** in unmittelbarer Nähe der Elektroden **30, 40** unter Verwendung der angegebenen Kupplungsreagenzien permanent immobilisiert.

Nachdem teilchenselektive Verbindungen auf dem Träger **20** und/oder den Elektroden **30, 40** unlösbar aufgebracht sind, trocknet man weitere Liganden und/oder biologisch aktive spezifische Bindungsmoleküle, die mit katalytisch aktiven Proteinen verknüpft sein können, in oder unmittelbar stromabwärts der Probenauftragszone **22** derart ein, daß sie im feuchten Zustand beweglich sind. Um sie in nativer Form zu erhalten bzw. leicht wieder zu mobilisieren, werden sie meist in einer Lösung aufgetragen, die Hilfsstoffe wie Polyhydroxy- bzw. Polyhydroxycarbonylverbindungen oder globuläre Proteine enthält.

Nun tränkt man das Flüssigkeitsaufnahmeelement **60** mit einer Lösung eines der genannten grenzflächenaktiven Stoffe, trocknet es und befestigt es mit einem Haftmittel auf dem Träger **20.** Nach den Ausführungsformen der Figuren 1 bis 4 ist es so anzubringen, daß der größte Teil der Leiterbahnen **42** überdeckt wird, die Elektroden **30, 40** und Kontakte **44** jedoch frei bleiben. Normalerweise liegt das Flüssigkeitsaufnahmeelement **60** satt auf der mit Leiterbahnen **42** versehenen Trägeroberfläche **20** auf. Es kann jedoch auch nur mit seinen Längsseiten auf dem Träger **20** befestigt werden, so daß zwischen ihm und dem Träger **20** ein Hohlraum verbleibt.

Als nächstes fixiert man die längliche Unterstützung **52** auf den Längskanten **24** des Trägers **20** und bringt dann die Abdeckung **54** an. Letztere ist mit der länglichen Unterstützung **52** und in einer Abwandlung auch mit dem Flüssigkeitsaufnahmeelement **60** fest verbunden. Man montiert sie so, daß sich unter einer ihrer Öffnungen **56** die Probenauftragszone **22** befindet. So erhält man eine Meßvorrichtung **10** mit Reaktions- und Detektionsraum **50,** Zwischenraum **70** und Flüssigkeitsaufnahmeelement **60.**

In einer bevorzugten Erweiterung hat man die längliche Unterstützung **52** und die Abdeckung **54** vor dem Montieren mit einer Lösung der grenzflächenaktiven Stoffe behandelt und getrocknet. Bei einer besonders leistungsfähigen Variante dieser Erweiterung beschichtet man die den Reaktions- und Detektionsraum **50** ausmachenden Bereiche von Abdeckung **54,** Unterstützung **52** und eventuell Träger **20** mit hydrophilen Stoffen und die vom Reaktions- und Detektionsraum **50** stromabwärts sowie stromaufwärts angeordneten Regionen mit hydrophoben Verbindungen.

In einer nicht gezeichneten Erweiterung der erfindungsgemäßen Meßvorrichtung **10** bringt man die beiden gegenüberliegenden Streifen der länglichen Unterstützung **52** in jeweils zweigeteilter Form also insgesamt vier Streifen auf den Träger **20** auf. Ein erster Streifen erstreckt sich vom stromaufwärtigen Ende des Trägers **20** bis zum Zwischenraum **70** und ein zweiter Streifen vom Zwischenraum **70** bis an die Kontakte **44.** Streifen drei und vier verlaufen parallel zu den ersten beiden Streifen auf der gegenüberliegenden Längskante **24** des Trägers **20.** Bei einer derartigen Bauweise kann man die Abdeckung **54** ausschließlich an der länglichen Unterstützung **52** befestigen und das Flüssigkeitsaufnahmeelement **60** so überdecken, daß zwischen ihm und der Abdeckung **54** ein Spalt verbleibt. Dies ist günstig, um dem mit Meßprobe und Substratlösung getränkten und deshalb aufgeblähten Flüssigkeitsaufnahmeelement 60 noch ausreichend Platz zu bieten.

Um eine Analytmenge in einer Probenflüssigkeit zu bestimmen, verbindet man die Meßvorrichtung **10** mit einem elektrischen Messgerät und trägt eine Meßprobe auf. Enthält sie den Analyten, bindet er sich bereits auf der Probenauftragszone **22** oder unmittelbar stromabwärts an dort vorhandene bewegliche biologisch aktive spezifische Bindungsmoleküle. Diese tragen jeweils ein katalytisch aktives Protein und bilden mit dem Analyten einen Komplex.

Dieser wandert mit der Probenlösung infolge Kapillarwirkung und aufgrund der mit Detergenzien beschichteten Meßvorrichtung **10** selbsttätig in den formstabilen Reaktions- und Detektionsraum **50.** Dort bindet er an immobilisierte Liganden oder an immobilisierte biologisch aktive spezifische Bindungsmoleküle, die entweder auf wenigstens einer Arbeitselektrode **40** oder in unmittelbarer Nähe zu ihr angeordnet sind. Die somit gebildete Sandwich-Anordnung besteht aus der beweglichen teilchenselektiven Verbindung, dem Analyt und der unbeweglichen teilchenselektiven Verbindung.

Überschüssige Probenlösung fließt stromabwärts in den Zwischenraum **70** und von dort in das Flüssigkeitsaufnahmeelement **60.** Dies geschieht jedoch nicht kontinuierlich, weil sich ein Gleichgewicht zwischen Sogwirkung des Flüssigkeitselements **60** einerseits und Rückhaltewirkung im Reaktions- und Detektionsraum **50** andererseits einstellt. Ist auf der Probenauftragszone **22** keine stromabwärts fließende Meßlösung mehr vorhanden, hält die Sogwirkung so lange an, bis es im Bereich des als Luftspalt ausgeführten Zwischenraums **70** zu einem Abreißen des Meßlösungsstroms kommt. Somit wird ein Teil der Probenlösung vom Flüsssigkeitsaufnahmeelement **60** aufgenommen, während ein weiterer definierter Volumenteil im Reaktions- und Detektionsraum **50** zurückbleibt. Dieser stets konstante Teil ist von der Größe des Zwischenraums **70** abhängig. Gibt man dessen Abmessungen in der Meßvorrichtung **10** konstruktiv vor, erreicht man, daß im formkonstanten Reaktions- und Detektionsraum **50** immer ein gleichbleibendes Meßvolumen vorhanden ist. Diese konstruktive Maßnahme erhöht die Genauigkeit einer quantitativen Analytbestimmung beachtlich und ist ein wesentlicher Teil der Erfindung. Dies gilt auch, wenn der Zwischenraum **70** sehr klein gewählt wird, also makroskopisch nicht vorhanden ist.

Nachdem der konstante Volumenteil der Meßlösung zwischen 5 und 600 Sekunden, idealerweise jedoch zwischen 20 und 60 Sekunden im Reaktions- und Detektionsteil **50** belassen wurde und dementsprechend der Analyt in beschriebener Weise ein Sandwich bilden konnte, trägt man auf die Probenauftragszone **22** in einem zweiten Schritt eine Flüssigkeit auf, die ein reaktionsspezifisches Substrat für wenigstens ein katalytisch aktives Protein enthält. Es handelt sich hierbei meist um eine gepufferte Lösung, deren Volumen wenigstens dreimal so groß ist wie das Volumen der Meßprobe. Hierdurch wird im Reaktions- und Detektionsraum **50** noch vorhandene Meßlösung ausgespült, strömt in den Zwischenraum **70** und wird vom Flüssigkeitsaufnahmeelement **60** aufgenommen. Gleichzeitig reagiert das reaktionsspezifische Substrat mit der Menge an katalytisch aktivem Protein, die in der Sandwich-Verbindung im Reaktions- und Detektionsraum **50** zurückgehalten wurde. Es bildet sich eine elektroaktive Substratform, die an der oder einer Arbeitselektrode **40** oxidiert oder reduziert, jedenfalls elektrochemisch umgesetzt wird. Dabei kommt es zu einer Strom-Spannungsänderung, die an einem Potentiostaten abzulesen ist. Die Größe der Änderung des elektrischen Signals ist ein Maß für die Menge an umgesetztem reaktionsspezifischem Substrat und somit für die Menge an gebundenem katalytisch aktivem Protein. Mithin ist sie ein Maß für die Menge an Sandwich-Verbindung an oder in der Nähe der Arbeitselektrode(n) **40** und zeigt somit die Menge an Analyt in der Meßlösung an.

In einer Ausgestaltung der Meßvorrichtung **10** setzt ein erstes katalytisch aktives Protein ein reaktionsspezifisches Substrat zu einem reaktionsspezifischen Substrat 2 um. Erst dieses reagiert mit einem zweiten katalytisch aktiven Protein zu einer elektroaktiven Substratform, die wie vorher beschrieben an wenigstens einer Arbeitselektrode **40** umgesetzt wird. Das zweite katalytisch aktive Protein ist in der Regel auf der Arbeitselektrode **40** oder in unmittelbarer Nachbarschaft zu ihr immobilisiert. In einer Abwandlung der Erfindung setzt man es jedoch der gepufferten Lösung des reaktionsspezifischen Substrats zu.

Bestimmt man die Menge eines Analyten mit der in Fig. 3 wiedergegebenen Meßvorrichtung **10** und verwendet man eine spezifische und eine unspezifische Arbeitselektrode **40', 40",** so erhält man zwei elektrische Signale. Das Signal der unspezifischen Arbeitselektrode **40"** muß dann von dem der spezifischen Elektrode **40'** subtrahiert werden, um für den jeweiligen Analyten zutreffende Werte zu erhalten.

Die Erfindung wird anhand der folgenden Ausführungsbeispiele erläutert, die jedoch nur einen Ausschnitt aller erfindungsgemäß abdeckbaren Kombinationen abbilden.

### Beispiel 1a: Herstellung einer Zweielektroden-Meßvorrichtung 10 gemäß Fig. 1

Ausgangsmaterial für die Arbeitselektrode **40** der Vorrichtung **10** ist eine wässrige Graphit-Masse (Deltaforge GP-157, Fa. Acheson, US), die 3,56 mg Ferrocen-Dicarbonsäure pro 1 g Graphit-Masse enthält. Diesem Gemisch setzt man eine 0,01 molare phosphatgepufferte Lösung von pH 7,5 zu, in der 25% (w/v) Phenylboronsäure und 9 g/l NaCl gelöst sind und vermengt alles zu einer Graphitpaste. Für alle Leiterbahnen **42** und Kontakte **44** verwendet man eine Graphitpaste, die keine Zusätze enthält (C2000802D2, Fa. Gwent Electronic Materials Ltd., UK). Die Referenzelektrode **30** stellt man mit einer klassischen Silber/Silber-Chlorid-Paste her (C61003D7, Fa. Gwent Electronic Materials Ltd., UK). Als Material für eine Isolierschicht wird ein dielektrisches Polymer (D2000222D2, Fa. Gwent Electronic Materials Ltd., UK) verwendet.

Zunächst druckt man die Leiterbahnen **42** und Kontakte **44** mittels Siebdrucktechnik wie in Fig. 1 gezeigt auf eine PVC-Folie (10 x 50 mm) auf und trocknet diese 5 min bei 100 °C. Anschließend wird mit der gleichen Technik die Isolierschicht aus dielektrischem Polymer zur Eingrenzung der Elektrodenflächen aufgetragen und bei 80 °C für 30 Minuten getrocknet. Dann bringt man in gleicher Art nacheinander die Pasten für die Arbeitselektrode **40** und die Referenzelektrode 30 auf und trocknet erstere 5 min bei 100 °C und letztere 10 min bei 80 °C.

Stromaufwärts trägt man danach auf den Träger 20 5 µl einer 0,3 molaren Mannit-Lösung auf, die anti-Hämoglobin A1c (monoklonal)-Glucose-Dehydrogenase Konjugat (anti-HbA1c-GDH-Konjugat) in einer Konzentration von 1 mg/ml enthält. Außerdem enthält diese Lösung 0,3 mmol/l eines aus Pferdeserum gewonnenen Proteins, das mit einer 80 prozentigen Ammoniumsulfatlösung ausgefällt wird und in reinem Wasser löslich ist. Den so beschichteten Träger **20** trocknet man bei 40 °C im Vakuum.

Als Flüssigkeitsaufnahmeelement **60** wird ein Faservlies (Schleicher & Schüll 0966, 8 x 25 mm) verwendet. Man tränkt es mit einer Lösung aus 3 g C₂₀H₃₇NaO₇S in 100 ml gereinigten Wassers, trocknet es im warmen Luftstrom und befestigt es auf dem Träger 20. Es wird so stromabwärts der Elektroden **30, 40** angebracht, daß es nicht unmittelbar an sie anstößt, sondern eine kleine Lücke oder der Zwischenraum **70** frei bleibt.

Auf die Längskanten **24** des Trägers **20** bringt man die längliche Unterstützung **52** auf und befestigt hierauf die aus Polyester bestehende 10 x 22 mm große Abdeckung **54.** Diese wird, wie in Fig. 1 zu sehen, so angebracht, daß ihre halbkreisförmige Aussparung **56** die Probenauftragszone **22** freigibt.

### Beispiel 1b: Bestimmung von Hämoglobin A1c (HbA1c)

Man stellt eine Substratlösung her, die aus 6,5 ml einer phosphatgepufferten Kochsalzlösung (PBS-Lösung), aus 3,25 ml einer phosphatgepufferten Kochsalz-Glucoselösung (PBS-Glucoselösung) und aus 0,25 ml einer Lösung von β-Nicotinamid-adenindinucleotid (NAD) besteht. Die PBS-Lösung enthält 0,12 mol/l Phosphat, 0,15 mol/l NaCl und hat einen pH-Wert von 7,6. Die PBS-Glucoselösung beinhaltet zusätzlich Glucose in einer Konzentration von 0,1 g/ml. Die NAD-Lösung hat eine Konzentration von 80 mg/ml.

Die in Beispiel 1a beschriebene Meßvorrichtung **10** schließt man an einen Potentiostaten an und gibt 10 µl HbA1c-Kontrollblut (Biocon) auf die Probenauftragszone **22** auf. Nach einer Inkubationszeit von 20 Sekunden werden 30 µl Substratlösung aufpipettiert und weitere 20 Sekunden inkubiert. Nun liest man am Potentiostaten eine zugehörige Strom- oder Spannungsänderung ab.

### Beispiel 1c: Bestimmung von Hämoglobin A1c (HbA1c) in einem Kontrollexperiment

In einem parallelen Experiment unterwirft man eine weitere Probe des verwendeten Kontrollblutes (Biocon) einem HbA1c-Mikrosäulentest (Biocon 11044).

### Beispiel 1d: Bestimmung der Gesamt-Hämoglobinmenge mit einer Pt-Elektrode

In einem weiteren Parallelexperiment taucht man in bekannter Weise eine Platinelektrode in eine Probe obiger Kontrollblutlösung, in der man zuvor ein Hexacyanoferrat-II-salz (Ferrocyanid) gelöst hat. Dieses wird vom vorhandenen Hämoglobin zu Hexacyanoferrat III (Ferricyanid) oxidiert und elektrochemisch an der Platinelektrode wieder zu Ferrocyanid reduziert. Das dabei generierte elektrische Signal zeigt die Gesamtmenge an Hämoglobin in der Kontrollblutprobe an.

**Beispiel 1e:** Bestimmung des Hämoglobin A1c (HbA1c) - Anteils an der Gesamt-Hämoglobinmenge

Zur Bestimmung des Hämoglobin A1c (HbA1c) - Anteils am insgesamt vorhandenen Hämoglobin setzt man die in Beispiel 1d bestimmte Gesamt-Hämoglobinmenge zu den Werten aus Beispiel 1b und 1c in Beziehung. Dabei ergeben sich folgende prozentuale Anteile:

| | |
|---|---|
| HbA1c-Anteil mit der erfindungsgemäßen Meßvorrichtung **10** bestimmt | 4,90 % |
| HbA1c-Anteil auf einer Mikrosäule (Biocon 11044) bestimmt | 4,40 % |

### Beispiel 2a: Herstellung einer anderen Zweielektroden-Meßvorrichtung 10 gemäß Fig. 1

Ausgangsmaterial für die Arbeitselektrode **40** dieser Vorrichtung **10** ist eine wässrige Graphit-Masse (Deltaforge GP-157, Fa. Acheson, US), die 3,56 mg Ferrocen-Dicarbonsäure pro 1 g Graphit-Masse enthält. Diesem Gemisch setzt man 250 µl einer handelsüblichen Lösung von Gelafusal^{®} (Stickstoff-Gehalt 0,9 g/100 ml) zu und vermengt alles zu einer Graphitpaste. Für alle Leiterbahnen **42** und Kontakte **44** verwendet man eine Graphitpaste, die keine Zusätze enthält (C2000802D2, Fa. Gwent Electronic Materials Ltd., UK). Die Referenzelektrode 30 stellt man mit einer klassischen Silber/Silber-Chlorid-Paste her (C61003D7, Fa. Gwent Electronic Materials Ltd., UK). Als Material für eine Isolierschicht wird ein dielektrisches Polymer (D2000222D2, Fa. Gwent Electronic Materials Ltd., UK) verwendet.

Zunächst druckt man die Leiterbahnen **42** und Kontakte **44** mittels Siebdrucktechnik wie in Fig. 1 gezeigt auf eine PVC-Folie (10 x 50 mm) auf und trocknet diese 5 min bei 100 °C. Anschließend wird mit der gleichen Technik die Isolierschicht aus dielektrischem Polymer zur Eingrenzung der Elektrodenflächen aufgetragen und bei 80°C für 30 Minuten getrocknet. Dann bringt man in gleicher Art nacheinander die Pasten für die Arbeitselektrode **40** und die Referenzelektrode **30** auf und trocknet erstere 5 min bei 100 °C und letztere 10 min bei 80°C.

Auf die Arbeitselektrode **40** bringt man nun ein biologisch aktives spezifisches Bindungsmolekül und ein katalytisch aktives Protein auf. Beide Verbindungen können sowohl aneinander gebunden als auch einzeln auf der Arbeitselektrode **40** immobilisiert vorliegen.

Zunächst stellt man eine 0,1 molare 2-(N-Morpholino-ethansulfonsäure) Pufferlösung (MES-Pufferlösung) her, die 0,15 mol/l NaCl enthält und einen pH von 4,7 besitzt. Dann bereitet man eine 10 prozentige (w/v) wässrige Lösung von (Ethyl-dimethylamino-propyl)-carbodiimid (EDC-Lösung) und eine 14 prozentige (w/v) Lösung von N-Hydroxysuccinimid-sulfonsäure-Natriumsalz frisch zu. Außerdem stellt man einen Kupplungspuffer mit einem pH von 7,5 her. Er enthält 0,1 mol/l Natriumdihydrogenphosphat, 0,1 mol/l Dinatriumhydrogenphosphat und 0,15 mol/l Natriumchlorid. Schließlich bereitet man eine Protein/Hexit-Lösung, die 5 g/l Albumin aus dem Serum vom Rind und 0,50 mol/l Sorbit enthält.

In 1 ml der MES-Pufferlösung werden 5 mg eines anti-Streptokokken-A-Antikörpers und 5 mg Glucoseoxidase gelöst. Zu dieser Lösung gibt man 1 ml EDC-Lösung und 1 ml der Succinimidlösung und füllt mit MES-Pufferlösung auf 5 ml auf. Die erhaltene Lösung läßt man während 15 Minuten auf einer rotierenden Vorrichtung inkubieren, wobei anti-Streptokokken-A und Glucoseoxidase in eine chemisch reaktive Form übergehen.

Man pipettiert 5 µl dieser Lösung und 5 µl Kupplungspuffer auf die Arbeitselektrode **40** und läßt 30 Minuten einwirken. Dann saugt man ab, bringt 5 µl der Protein/Hexit-Lösung auf die Arbeitselektrode **40** auf und trocknet den Träger **20** samt Elektroden **30, 40** während 1 h Stunde bei 30 °C bis 50 °C im Vakuum.

Stromaufwärts trägt man danach auf den Träger **20** 5 µl einer 0,3 molaren alpha-D-Glucopyranosyl-beta-D-Fructofuranosid-Lösung auf, die Kaninchen anti-Streptokokken-A-Peroxidase Konjugat (anti-Strep-A-POD-Konjugat) in einer Konzentration von 1 mg/ml enthält. Außerdem enthält diese Lösung 0,3 mmol/l eines aus Pferdeserum gewonnen Proteins, das mit einer 80 prozentigen Ammoniumsulfatlösung ausgefällt wird und in reinem Wasser löslich ist. Den so beschichteten Träger **20** trocknet man bei 40 °C im Vakuum.

Als Flüssigkeitsaufnahmeelement **60** wird ein Faservlies (Schleicher & Schüll 0966, 10 x 25 mm) verwendet. Man tränkt es mit einer Lösung aus 3 g C₂₀H₃₇NaO₇S in 100 ml gereinigten Wassers, trocknet es im warmen Luftstrom und befestigt es auf dem Träger **20.** Es wird so stromabwärts der Elektroden **30, 40** angebracht, daß es nicht unmittelbar an sie anstößt, sondern eine Lücke oder der Zwischenraum **70** frei bleibt.

Auf die Längskanten **24** des Trägers **20** bringt man die längliche Unterstützung **52** auf und befestigt hierauf die aus Polyester bestehende 10 x 22 mm große Abdeckung **54.** Diese wird, wie in Fig. 1 zu sehen, so angebracht, daß ihre halbkreisförmige Aussparung **56** die Probenauftragszone **22** freigibt.

### Beispiel 2b: Bestimmung von Streptokokken A Antigen

Es wird eine Nitritlösung hergestellt, die 1 mol/l Natriumnitrit enthält. Dann präpariert man eine 0,1 molare Glycinlösung, die mit Salzsäure auf einen pH-Wert von 2,5 gebracht wird. Außerdem bereitet man eine 0,2 molare Tris-Pufferlösung mit einem pH-Wert von 9 und eine wässrige Substratlösung, die 20 mmol/l β-D-Glucose und 3 mmol/l Kaliumjodid enthält.

Von zwei Patienten werden je zwei Rachenabstriche genommen:
Patient 1: Alter 7 Jahre, Geschlecht: männlich, Grund des Arztbesuches: Erkältungssymptome, Infekt der Luftwege;
Patient 2: Alter 5 Jahre, Geschlecht: weiblich; Grund des Arztbesuches: Halsschmerzen

Man mischt 200 µl der Glycinlösung mit 200 µl der Nitritlösung in einem Extraktionsgefäß. In dieses taucht man den ersten Abstrich eines Patienten und extrahiert ihn zwei Minuten. Dann gibt man 200 µl Tris-Pufferlösung hinzu. 10 µl dieser Extraktionslösung werden auf die Probenauftragszone **22** der mit einem Potentiostaten verbundenen Meßvorrichtung 10 pipettiert und 20 Sekunden inkubiert. Dann trägt man 30 µl der Substratlösung auf und inkubiert nochmals 20 Sekunden. Der mit dem Potentiostaten gemessene Strom zeigt an, daß im Abstrich des Patienten 1 kein Streptokokken-A Antigen, im Abstrich von Patient 2 jedoch Streptokokken-A Antigen vorhanden ist. Dabei entspricht das erhaltene elektrische Signal etwa 4 × 10⁵ KBE/ml (= koloniebildende Einheiten/ml).

### Beispiel 2c: Bestimmung von Streptokokken A Antigen in einem Kontrollexperiment

Abstrich 2 beider Patienten wird zwecks kultureller Anzucht von Streptokokken-A Stämmen in Röhrchen mit Transportmedium überführt. Die Kultivierung der Stämme erfolgt auf Schaedler-Blutagar über 24 Stunden bei 37 °C anaerob. Die qualitative Auswertung ergibt in Übereinstimmung mit Beispiel 2b, daß im Abstrich von Patient 1 kein Streptokkoken-A Antigen vorhanden ist, der Abstrich von Patient 2 jedoch Streptokokken-A Antigen enthält. Das Ergebnis stützt sich auf die Bestätigung von beta-hämolysierenden Streptokokken der serologischen Gruppe A mit einem Streptokokken-Identifizierungstest zum Nachweis dieser Bakterien (Oxoid). Die Zählung der Streptokokkenkolonien erfolgt nach S. Isaac, D. Jennings: Kultur von Mikroorganismen, Spektrum Akademischer Verlag Heidelberg, Berlin, Oxford, S. 87 und ergibt 2 x 10⁵ koloniebildende Einheiten pro ml (KBE/ml).

### Beispiel 3a: Herstellung einer Dreielektroden-Meßvorrichtung 10 gemäß Fig. 3

Ausgangsmaterial für die Arbeitselektroden **40** dieser Vorrichtung **10** ist eine wässrige Graphit-Masse (Deltaforge GP-157, Fa. Acheson, US), die 3,56 mg Ferrocen-Dicarbonsäure pro 1 g Graphit-Masse enthält. Diesem Gemisch setzt man 125 µl einer 20 prozentigen (w/v) Lösung von Poly-Aminopentandisäure in Wasser zu und vermengt alles zu einer Graphitpaste. Für alle Leiterbahnen **42** und Kontakte **44** verwendet man eine Graphitpaste, die keine Zusätze enthält (C2000802D2, Fa. Gwent Electronic Materials Ltd., UK). Die Referenzelektrode **30** stellt man mit einer klassischen Silber/Silber-Chlorid-Paste her (C61003D7, Fa. Gwent Electronic Materials Ltd., UK). Als Material für eine Isolierschicht wird ein dielektrisches Polymer (D2000222D2, Fa. Gwent Electronic Materials Ltd., UK) verwendet.

Zunächst druckt man die Leiterbahnen **42** und Kontakte **44** mittels Siebdrucktechnik wie in Fig. 3 gezeigt auf eine PVC-Folie (10 x 50 mm) auf und trocknet diese 5 min bei 100 °C. Anschließend wird mit der gleichen Technik die Isolierschicht aus dielektrischem Polymer zur Eingrenzung der Elektrodenflächen aufgetragen und bei 80°C für 30 Minuten getrocknet. Dann bringt man in gleicher Art nacheinander die Pasten für die Arbeitselektrode **40** und die Referenzelektrode **30** auf und trocknet erstere 5 min bei 100 °C und letztere 10 min bei 80°C.

Man stellt eine 0,1 molare 2-(N-Morpholino-ethansulfonsäure) Pufferlösung (MES-Pufferlösung) her, die 0,15 mol/l NaCl enthält und einen pH von 4,7 besitzt. Dann bereitet man eine 10 prozentige (w/v) wässrige Lösung von (Ethyl-dimethyl-aminopropyl)-carbodiimid (EDC-Lösung) und eine 14 prozentige (w/v) Lösung von N-Hydroxysuccinimid-sulfonsäure-Natriumsalz frisch zu. Außerdem stellt man einen Kupplungspuffer mit einem pH von 7,5 her. Er enthält 0,1 mol/l Natriumdihydrogenphosphat, 0,1 mol/l Dinatriumhydrogenphosphat und 0,15 mol/l Natriumchlorid.

Monoklonale Maus anti-Chlamydia trachomatis Antikörper und Glucoseoxidase werden in soviel Kupplungspuffer gelöst, daß er eine Antikörperkonzentration von 2 mg/ml und eine Glucoseoxidaseaktivität von 150 U/ml aufweist (Kupplungslösung). Außerdem stellt man eine Lösung von 2 mg/ml unspezifischem Maus-Immunglobulin G und Glucoseoxidase mit einer Aktivität von 150 U/ml in Kupplungspuffer her (Maus-IgG-Lösung). Schließlich bereitet man eine Protein/Hexit-Lösung, die 5 g/l Albumin aus dem Serum vom Rind und 0,50 mol/l Sorbit enthält.

4 ml der MES-Pufferlösung werden mit 1 ml der EDC-Lösung und 1 ml der Hydroxysuccinimid-sulfonsäure-Natriumsalz-Lösung vermischt. Von dem so erhaltenen Lösungsgemisch pipettiert man 5 µl auf die spezifische und die unspezifische Arbeitselektrode **40** und inkubiert während 15 Minuten, um deren Oberflächen zu aktivieren.

Auf die aktivierte spezifische Arbeitselektrode **40'** gibt man 5 µl der Kupplungslösung, die monoklonale Maus anti-Chlamydia trachomatis Antikörper und Glucoseoxidase enthält. Auf die aktivierte unspezifische Arbeitselektrode **40"** wird 5 µl der Maus-lgG-Lösung aufgetragen. Nach einer Inkubationszeit von 30 Minuten saugt man ab, pipettiert 5 µl Protein/Hexitlösung auf die Arbeitselektroden **40', 40"** und trocknet eine Stunde bei 30 °C bis 50 °C im Vakuum.

Stromaufwärts trägt man danach auf den Träger 20 5 µl einer 0,5 molaren Sorbit-Lösung auf, die anti-Chlamydia trachomatis-Peroxidase-Konjugat in einer Konzentration von 1 mg/ml enthält. Außerdem enthält diese Lösung Albumin aus dem Serum von Rind in einer Konzentration von 5 g/l. Den so beschichteten Träger **20** trocknet man bei 40 °C im Vakuum.

Als Flüssigkeitsaufnahmeelement **60** wird ein Faservlies (Schleicher & Schüll 0966, 10 x 25 mm) verwendet. Man tränkt es mit einer Lösung aus 3 g C₂₀H₃₇NaO₇S in 100 ml gereinigten Wassers, trocknet es im warmen Luftstrom und befestigt es auf dem Träger **20.** Es wird so stromabwärts der Elektroden **30, 40** angebracht, daß es nicht unmittelbar an sie anstößt, sondern eine kleine Lücke oder der Zwischenraum **70** frei bleibt.

Auf die Längskanten **24** des Trägers **20** bringt man die längliche Unterstützung **52** auf und befestigt hierauf die aus Polyester bestehende 10 x 22 mm große Abdeckung **54.** Diese wird, wie in Fig. 3 zu sehen, so angebracht, daß ihre halbkreisförmige Aussparung **56** die Probenauftragszone **22** freigibt.

### Beispiel 3b: Bestimmung von Chlamydia trachomatis Antigen

BGM-Zellen der Affennieren-Fibroblasten- Zelllinie (10⁶ Zellen) werden in Kulturflaschen (25 cm²) in einem Eagle Medium mit 10 prozentigem fetalem Kälberserum zum dichten Zellrasen angezüchtet. Nach Absaugen des Mediums wird mit 1 ml Chlamydia trachomatis-Suspension beimpft. Es handelt sich bei dem Chlamydia trachomatis Erreger um einen L2 Stamm, der vom Institut für Medizinische Mikrobiologie der Friedrich-Schiller-Universität Jena erhalten und auf einen Gehalt von 1 x 10⁶ IFU/ml (= infektiöse Einheiten/ml) eingestellt wurde. Die Suspension hält man zur vollständigen Infektion 2 Stunden bei 37 °C und bebrütet sie dann 3 Tage bei gleicher Temperatur unter einer 5 prozentigen CO₂ Atmosphäre. Die erhaltenen Zellen werden durch Ultraschallbehandlung aufgeschlossen und dienen als Positivkontrolle.

Man bereitet eine wässrige Substratlösung zu, die 20 mmol/l β-D-Glucose und 3 mmol/l Kaliumjodid enthält.

10 µl der Chlamydia trachomatis-Positivkontrolle werden auf die Probenauftragszone **22** der mit einem Potentiostaten verbundenen Meßvorrichtung **10** pipettiert und 20 Sekunden inkubiert. Dann trägt man 30 µl der Substratlösung auf und inkubiert nochmals 20 Sekunden. Der Potentiostat zeigt ein elektrisches Signal an, das gemäß Eichkurve einem Chlamydia trachomatis Antigen Titer von 1 : 256 entspricht. Unter Titer versteht man die Endverdünnung einer geometrischen Verdünnungsreihe, bei der gerade noch eine positive Reaktion angezeigt wird.

### Beispiel 3c: Bestimmung des Gehaltes von Chlamydia trachomatis Antigen in einem Kontrollexperiment

Mit einem Chlamydien-Antigen-ELISA, "Chlamidia Celisa", Cellabs Diagnostics PTY. Australien" bestimmt man einen Titer von 1 : 256.

Die Erfindung ist nicht auf eine der vorbeschriebenen Ausführungsformen beschränkt, sondern in vielfältiger Weise abwandelbar.

So läßt sich der Träger **20** und die Abdeckung **54** auch aus anorganischen Materialien wie Glas, Keramik oder Graphit ausbilden.

Außerdem kann die Meßvorrichtung **10** auch nach dem Prinzip eines kompetitiven Bindungsassays aufgebaut sein. Hierzu bringt man keine im feuchten Zustand beweglichen, biologisch aktiven spezifischen Bindungsmoleküle auf den Träger **20** auf, sondern beschichtet ihn mit einem im feuchten Zustand beweglichen Konjugat aus Analyt und katalytisch aktivem Protein. Solange der gesuchte Analyt in der Meßprobe nicht vorhanden ist, strömt das Konjugat stromabwärts und bindet vollständig an die auf wenigstens einer Arbeitselektrode **40** oder in unmittelbarer Nachbarschaft zu ihr immobilisierten biologisch aktiven spezifischen Bindungsmoleküle. Ist jedoch Analyt in der Meßprobe enthalten, konkurriert er mit dem Konjugat um Bindungsstellen der immobilisierten biologisch aktiven spezifischen Bindungsmoleküle. Dementsprechend strömt ein nicht gebundener Teil des Konjugats weiter in den Zwischenraum **70** und von dort in das Flüssigkeitsaufnahmeelement **60.** Weil weniger Konjugat im Bereich der Arbeitselektrode **40** fixiert ist, kann weniger Substrat umgesetzt werden, was zu einem kleineren elektronischen Signal an der Arbeitselektrode **40** führt. Die Abnahme der Signalhöhe ist ein quantitatives Maß für die Menge an Analyt in der Meßprobe.

Man erkennt, daß eine Meßvorrichtung **10** zum Erfassen und zur Bestimmung der Konzentration wenigstens eines Analyten in einem flüssigen Medium aus einem polymeren Träger **20** mit stromaufwärtig eingetrockneten biologisch aktiven spezifischen Bindungsmolekülen besteht. Auf ihm befindet sich stromabwärts ein Mehrfachelektrodensatz aus wenigstens einer Referenzelektrode **30** und aus wenigstens einer Arbeitselektrode **40.** Letztere ist mit biologisch aktiven spezifischen Bindungsmolekülen versehen. Der Träger **20** verfügt über eine Probenauftragszone **22** an die sich stromabwärts ein durchströmbarer unverformbarer Reaktions- und Detektionsraum **50,** ein in seinen Abmessungen variabel dimensionierter Zwischenraum **70** und ein Flüssigkeitsaufnehmeelement **60** anschließen. Für eine qualitative und quantitative Analytbestimmung werden zwei flüssige Medien nacheinander auf die Probenauftragszone **22** aufgetragen, nämlich eine Probenlösung und eine Substratlösung. Während die erste den gesuchten Analyten enthalten kann, sorgt die zweite für die Ausbildung eines elektrischen Signals. Infolge der geometrisch regulären und durch fluide Medien nicht verformbaren Ausführung von Reaktions- und Detektionsraum **50** und Zwischenraum 70 lassen sich exakte Messungen durchführen.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritte, können sowohl für sich als auch in den verschiedensten Kombinationen erfindungswesentlich sein.

### Bezugszeichenliste

- 10: Meßvorrichtung
- 20: Träger
- 22: Probenauftragszone
- 24: Längskante
- 30: Referenzelektrode
- 40, 40', 40": Arbeitselektrode
- 42: Leiterbahn
- 44: Kontakt
- 50: Reaktions- und Detektionsraum
- 52: längliche Unterstützung
- 54: Abdeckung
- 56: Aussparung/Öffnung
- 60: Flüssigkeitsaufnehmelement
- 70: Zwischenraum

## Patentansprüche

1. Vorrichtung zum Erfassen und/oder zur Bestimmung der Konzentration wenigstens eines in einem flüssigen Medium vorhandenen Analyten, bestehend
a) aus einem streifenförmigen Träger **(20)** mit zwei Längskanten **(24),** auf dem ein gedruckter Mehrfach-Elektrodensatz aus wenigstens einer Referenzelektrode **(30)** und aus wenigstens einer Arbeitselektrode **(40)** aufgebracht ist, [und]
wobei jede Arbeitselektrode **(40)** und jede Referenzelektrode **(30)** mit einer ihr zugeordneten Leiterbahn **(42)** und jede Leiterbahn **(42)** mit einem ihr zugeordneten, an ein elektrisches Meßgerät anschließbaren Kontakt **(44)** leitend verbunden ist,
b) aus Liganden, die geeignet sind, ein biologisch aktives spezifisches Bindungsmolekül oder den Analyten zu binden
c) und aus einer an einem Ende des Trägers **(20)** ausgebildeten Probenauftragszone (22),
**dadurch gekennzeichnet,**
a) **daß** die Vorrichtung einen starren, kontinuierlich entlang des streifenförmigen Trägers **(20)** durchströmbaren Reaktions- und Detektionsraum **(50)** besitzt, der durch eine auf den Längskanten **(24)** des Trägers **(20)** aufgebrachte längliche Unterstützung **(52)** mit einer darauf angeordneten Abdeckung **(54)** gebildet ist,
b) **daß** stromabwärts des Reaktions- und Detektionsraums **(50)** ein Flüssigkeitsaufnahmeelement **(60)** angeordnet ist,
c) **daß** die längliche Unterstützung **(52)** zweigeteilt ist und in Form von zwei Streifen auf den beiden Längskanten **(24)** des Trägers **(20)** permanent haftend aufliegt und
d) **daß** die Streifen nur auf einem Teil der beiden Längskanten **(24)** des Trägers **(20)** aufliegen, wobei zwischen den Streifen und dem Flüssigkeitsaufnahmeelement (60) ein Zwischenraum **(70)** besteht, von dem aus sich die Streifen auf den Längskanten **(24)** stromaufwärts erstrecken.

2. Vorrichtung nach Anspruch 1, **dadurch gekennzeichnet, daß** der Träger **(20)** als selbsttragende Fließfläche für das flüssige Medium ausgebildet ist.

3. Vorrichtung nach Anspruch 1 oder 2, **dadurch gekennzeichnet, daß** der Träger **(20)** einstückig ist und aus einem polymeren, nichtleitenden, vorzugsweise rechteckigen Material besteht.

4. Vorrichtung nach einem der Ansprüche 1 bis 3, **dadurch gekennzeichnet, daß** auf dem Träger **(20)** Liganden und/oder biologisch aktive spezifische Bindungsmoleküle aufgebracht sind.

5. Vorrichtung nach Anspruch 4, **dadurch gekennzeichnet, daß** die Liganden und/oder die biologisch aktiven spezifischen Bindungsmoleküle auf dem Träger **(20)** immobilisiert sind.

6. Vorrichtung nach einem der Ansprüche 1 bis 5, **dadurch gekennzeichnet, daß** der Träger **(20)** mit einer Grundbeschichtung ausgestattet ist, die aus Proteinen aus der Gruppe der tierischen oder menschlichen Albumine und/oder aus der Gruppe der Globuline und/oder aus der Gruppe der Glykoproteine besteht.

7. Vorrichtung nach einem der Ansprüche 1 bis 6, **dadurch gekennzeichnet, daß** der Träger **(20)** mit einer Grundbeschichtung versehen ist, die aus Polypeptiden, beispielsweise Polyaminosäuren und/oder aus Abbauprodukten von Proteinen vorzugsweise des Stütz- und Bindegewebes besteht.

8. Vorrichtung nach einem der Ansprüche 1 bis 7, **dadurch gekennzeichnet, daß** die Probenauftragszone **(22)** oder wenigstens ein Teil von ihr mit grenzflächenaktiven Stoffen behandelt ist und die Fähigkeit besitzt, das flüssige Medium aufzusaugen.

9. Vorrichtung nach einem der Ansprüche 1 bis 8, **dadurch gekennzeichnet, daß** auf der Probenauftragszone **(22)** ein maschenförmiges oder poröses, vorzugsweise mit grenzflächenaktiven Stoffen behandeltes Material aufgebracht ist.

10. Vorrichtung nach einem der Ansprüche 1 bis 9, **dadurch gekennzeichnet, daß** wenigstens eine der Elektroden **(30, 40)** sowie die ihr jeweils zugeordnete Leiterbahn **(42)** und der ihr jeweils zugeordnete Kontakt **(44)** aus Gold, Silber, Platin, Nickel, Palladium, Titan, Kupfer oder Kohlenstoff bestehen.

11. Vorrichtung nach einem der Ansprüche 1 bis 10, **dadurch gekennzeichnet, daß** wenigstens eine der Elektroden **(30, 40)** aus einer getrockneten Graphitpaste besteht oder eine aus Metallpasten und/oder Metallsalzpasten durch Trocknung herstellbare Elektrode ist.

12. Vorrichtung nach einem der Ansprüche 1 bis 11, **dadurch gekennzeichnet, daß** wenigstens eine Arbeitselektrode **(40)** Liganden und/oder biologisch aktive spezifische Bindungsmoleküle trägt, beispielsweise an ihrer Oberfläche.

13. Vorrichtung nach einem der Ansprüche 1 bis 12, **dadurch gekennzeichnet, daß** auf wenigstens einer weiteren Arbeitselektrode **(40)** reaktionsunspezifische Bindungsmoleküle angeordnet und/oder immobilisiert sind.

14. Vorrichtung nach einem der Ansprüche 1 bis 13, **dadurch gekennzeichnet, daß** die Liganden als Lösung mit einer Konzentration von 0,0007 bis 0,7 mol/l, vorzugsweise von 0,035 bis 0,35 mol/l aufgetragen sind.

15. Vorrichtung nach einem der Ansprüche 1 bis 14, **dadurch gekennzeichnet, daß** die Liganden aus Ribonukleinsäuren oder Desoxyribonukleinsäuren gebildet sind.

16. Vorrichtung nach einem der Ansprüche 1 bis 15, **dadurch gekennzeichnet, daß** die Liganden aus Aminocarbonsäuren, vorzugsweise Diaminocarbonsäuren bestehen.

17. Vorrichtung nach Anspruch 16, **dadurch gekennzeichnet, daß** die Liganden aus höhermolekularen, homodet oder heterodet peptidartig verknüpften Aminocarbonsäuren der Zusammensetzung R-CH(NH₂)-(CH₂)ₙ-COOH bestehen, beispielsweise aus Polyaminocarbonsäuren, wobei der Rest R für ein Proton oder vorzugsweise für eine Amino-, Imino-, Hydroxyl-, Thiol-, Hydroxyalkyl-, Aminoalkyl- oder Carboxyalkyl-Gruppe steht und n einen Wert von 0 bis 6, vorzugsweise 0 annimmt.

18. Vorrichtung nach einem der Ansprüche 1 bis 17, **dadurch gekennzeichnet, daß** die Liganden aus Proteiden bzw. aus Proteinen bestehen, die vorzugsweise aus dem Bacterium Streptomyces avidinii stammen.

19. Vorrichtung nach einem der Ansprüche 1 bis 18, **dadurch gekennzeichnet, daß** beispielsweise zum Nachweis von HbA1c wenigstens ein Ligand eine Phenylboronsäure ist.

20. Vorrichtung nach einem der Ansprüche 1 bis 19, **dadurch gekennzeichnet, daß** die biologisch aktiven spezifischen Bindungsmoleküle Bindungsstellen für wenigstens einen Liganden besitzen oder an ihn gebunden sind.

21. Vorrichtung nach einem der Ansprüche 1 bis 20, **dadurch gekennzeichnet, daß** die biologisch aktiven spezifischen Bindungsmoleküle für die Bindung eines Analyten wenigstens eine spezifische Bindungsregion aufweisen.

22. Vorrichtung nach einem der Ansprüche 1 bis 21, **dadurch gekennzeichnet, daß** ein katalytisch aktives Protein an die biologisch aktiven spezifischen Bindungsmoleküle und/oder die Liganden vorzugsweise kovalent gebunden ist oder daß mehrere katalytisch aktive Proteine an die biologisch aktiven spezifischen Bindungsmoleküle und/oder die Liganden vorzugsweise kovalent gebunden sind.

23. Vorrichtung nach einem der Ansprüche 1 bis 22, **dadurch gekennzeichnet, daß** die Streifen aus einem Polymerwerkstoff und/oder aus einem Schmelzkleber bestehen und die gleiche Dicke wie das Flüssigkeitsaufnahmeelement **(60)** besitzen.

24. Vorrichtung nach einem der Ansprüche 1 bis 23, **dadurch gekennzeichnet, daß** die Abdeckung **(54)** aus einem polymeren, vorzugsweise rechteckigen Material besteht und mit der länglichen Unterstützung (52) derart verbunden ist, daß sie den Träger **(20)** sowie den Mehrfach-Elektrodensatz und das Flüssigkeitsaufnahmeelement **(60)** bedeckt.

25. Vorrichtung nach einem der Ansprüche 1 bis 24, **dadurch gekennzeichnet, daß** die Abdeckung **(54)** nur einen oder mehrere Teile des Trägers **(20)** bedeckt.

26. Vorrichtung nach einem der Ansprüche 1 bis 25, **dadurch gekennzeichnet, daß** die Abdeckung **(54)** an ihrer stromaufwärtigen Seite wenigstens eine schlitz- und/oder halbkreisförmige beziehungsweise vieleckige Aussparung **(56)** und/oder Öffnung **(56)** besitzt, die so angeordnet ist, daß die Probenauftragszone **(22)** des Trägers **(20)** mit flüssigem Medium beschickbar ist.

27. Vorrichtung nach einem der Ansprüche 1 bis 26, **dadurch gekennzeichnet, daß** das Flüssigkeitsaufnahmeelement **(60)** an einer dem Träger (20) zugewandten Seite der Abdeckung **(54)** haftend befestigt ist.

28. Vorrichtung nach einem der Ansprüche 1 bis 27, **dadurch gekennzeichnet, daß** die Abdeckung **(54)** oder wenigstens ein Teil von ihr mit grenzflächenaktiven Stoffen behandelt ist.

29. Vorrichtung nach einem der Ansprüche 1 bis 28, **dadurch gekennzeichnet, daß** die dem Träger **(20)** zugewandte Seite der Abdeckung **(54)** im Bereich des Mehrfach-Elektrodensatzes, insbesondere oberhalb der Arbeitselektrode(n) **(40)** und oberhalb der Referenzelektrode **(30)** hydrophile Oberflächeneigenschaften besitzt und am Ort der Probenauftragszone **(22)** zumindest teilweise eine hydrophobe Oberfläche aufweist.

30. Vorrichtung nach einem der Ansprüche 1 bis 29, **dadurch gekennzeichnet, daß** der Reaktions- und Detektionsraum **(50)** in stromabwärtiger Richtung und in stromaufwärtiger Richtung offen ist und der Zwischenraum **(70)** stromabwärts an ihn angrenzt.

31. Vorrichtung nach einem der Ansprüche 1 bis 30, **dadurch gekennzeichnet, daß** das Flüssigkeitsaufnahmeelement **(60)** auf dem Träger **(20)** stromabwärts von der Probenauftragszone **(22)** beabstandet angeordnet ist und die Leiterbahnen **(42)** überdeckt, beispielsweise fest auf ihnen aufliegt.

32. Vorrichtung nach einem der Ansprüche 1 bis 31, **dadurch gekennzeichnet, daß** das Flüssigkeitsaufnahmeelement **(60)** aus einem saugfähigen porösen fibrillären Material besteht und auf dem Träger **(20)** haftend befestigt ist.

33. Vorrichtung nach Anspruch 32, **dadurch gekennzeichnet, daß** das poröse Material aus wenigstens einer Faserschicht oder aus wenigstens einem Gewebe aufgebaut ist, welche(s) aus Cellulosefasern oder Glasfasern oder Gemischen daraus besteht bzw. sich aus organischen Polymeren zusammensetzt.

34. Vorrichtung nach einem der Ansprüche 1 bis 33, **dadurch gekennzeichnet, daß** das Flüssigkeitsaufnahmeelement **(60)** mit grenzflächenaktiven Stoffen behandelt ist, vorzugsweise mit solchen, die hydrophile Eigenschaften haben.

35. Vorrichtung nach einem der Ansprüche 8 bis 34, **dadurch gekennzeichnet, daß** die grenzflächenaktiven Stoffe hydrophile Gruppen, beispielsweise - COOMe, -OSO₃Me, -SO₃Me, -NH₂, =NH, -NR₃⁺ und hydrophobe Alkylketten mit 10 bis 18 C-Atomen oder Alkylaryl-Reste enthalten.

36. Vorrichtung nach einem der Ansprüche 8 bis 35, **dadurch gekennzeichnet, daß** als grenzflächenaktive Stoffe vorzugsweise Substanzen mit den Summenformeln C₂₀H₃₇NaO₇S oder C₁₈H₂₉NO₃S verwendet werden.

37. Vorrichtung nach einem der Ansprüche 3 bis 36, **dadurch gekennzeichnet, daß** das polymere Material ein Polyethylen, ein Polystyrol, ein Polyurethan, ein Polyvinylacetat, ein Polyester, beispielsweise Polyethylenterephthalat, ein Epoxyharz, ein Methacryl-Polymer, ein Polycarbonat, ein Polyvinylchlorid oder ein Copolymer der genannten Verbindungen ist.

38. Vorrichtung nach einem der Ansprüche 3 bis 37, **dadurch gekennzeichnet, daß** das polymere Material eine bevorzugt aus Polyester, Polycarbonat oder Polyvinylchlorid gefertigte Folie ist.

39. Verfahren zum Herstellen einer Vorrichtung **(10)** zum Erfassen und zur Bestimmung der Konzentration wenigstens eines in einem flüssigen Medium gelösten Analyten nach einem der Ansprüche 1 bis 38, **dadurch gekennzeichnet,**
a) **daß** ein Elektrodenmaterial zu einer Paste verarbeitet und diese Paste auf einen Träger **(20)** derart aufgebracht wird, daß ein Mehrfach-Elektrodensatz aus wenigstens einer Arbeitselektrode **(40)** und aus wenigstens einer Referenzelektrode **(30)** sowie aus daran angeschlossenen Leiterbahnen **(42)** und Kontakten **(44)** gebildet wird,
b) **daß** der mit dem Mehrfach-Elektrodensatz versehene Träger **(20)** bei erhöhter Temperatur getrocknet wird,
c) **daß** auf den Träger **(20)** Liganden und/oder biologisch aktive spezifische Bindungsmoleküle und/oder katalytisch aktive Proteine aufgebracht werden, und zwar im Bereich des Mehrfach-Elektrodensatzes oder in einem Bereich, der stromaufwärts des Mehrfach-Elektrodensatzes angeordnet ist und
d) **daß** auf den so erhaltenen Träger **(20)** eine längliche Unterstützung **(52),** eine Abdeckung **(54)** und ein Flüssigkeitsaufnahmeelement **(60)** so befestigt werden, daß Abdeckung **(54)** und Träger **(20)** zueinander einen Abstand haben.

40. Verfahren nach Anspruch 39, **dadurch gekennzeichnet, daß** die Paste Liganden und/oder biologisch aktive spezifische Bindungsmoleküle und/oder katalytisch aktive Proteine enthält und mittels Siebdrucktechnik aufgetragen wird.

41. Verfahren nach einem der Ansprüche 39 oder 40, **dadurch gekennzeichnet, daß** wenigstens eine der getrockneten Elektroden **(40)** mit Liganden und/oder biologisch aktiven, spezifischen Bindungsmolekülen und/oder katalytisch aktiven Proteinen vorzugsweise unter Einsatz von Vernetzungsmolekülen oder Kupplungsreagenzien beschichtet wird,

42. Verfahren nach einem der Ansprüche 39 bis 41, **dadurch gekennzeichnet, daß** wenigstens eine der getrockneten Elektroden **(40)** mit Liganden und/oder reaktionsunspezifischen Bindungsmolekülen vorzugsweise unter Einsatz von Vernetzungsmolekülen oder Kupplungsreagenzien beschichtet wird.

43. Verfahren nach einem der Ansprüche 39 bis 42 **dadurch gekennzeichnet, daß** das Flüssigkeitsaufnahmeelement (60) mit einer Lösung eines grenzflächenaktiven Stoffes behandelt und getrocknet wird, wobei dieser Stoff in einer bevorzugten Ausführungsform eine Verbindung der Summenformel C₂₀H₃₇NaO₇S oder C₁₈H₂₉NO₃S ist und in einer Konzentration von 0,001 bis 5 % w/v, vorzugsweise von 0,1 bis 3 % w/v eingesetzt wird.

44. Verfahren zum Erfassen sowie zur Bestimmung der Konzentration wenigstens eines in einem flüssigen Medium gelösten Analyten mit einer Vorrichtung (10), die aus wenigstens einer Arbeitselektrode **(40)** und wenigstens einer Referenzelektrode **(30)** besteht, nach einem der Ansprüche 1 bis 43, **dadurch gekennzeichnet,**
a) **daß** eine definierbare Menge des flüssigen Mediums auf eine Probenauftragszone **(22)** der Vorrichtung **(10)** aufgebracht wird,
b) **daß** eine vorgebbare Zeit abgewartet wird, um eine Wechselwirkung des/der Analyten mit Liganden und/oder biologisch aktiven spezifischen Bindungsmolekülen und/oder katalytisch aktiven Proteinen stattfinden zu lassen,
c) **daß** auf die Probenauftragszone **(22)** eine Lösung aufgebracht wird, die wenigstens ein für ein katalytisch aktives Protein oder für mehrere katalytisch aktiven Proteine reaktionsspezifisches Substrat enthält,
d) **daß** die Vorrichtung **(10)** mit einem elektrischen Meßgerät verbunden wird und
e) **daß** nach einer Inkubationszeit auf dem an die Vorrichtung **(10)** angeschlossenen Meßgerät für jeden zu bestimmenden Analyten ein elektrisches Signal abgelesen und diesem Signal anhand einer Eichkurve eine entsprechende Konzentration des jeweiligen Analyten zugeordnet wird.

45. Verfahren nach Anspruch 44, **dadurch gekennzeichnet, daß** die definierbare Menge 0,01 bis 20 µl, vorzugsweise 1 bis 10 µl beträgt und daß die vorgebbare Zeit zwischen 5 und 600 Sekunden, vorzugsweise zwischen 10 und 120 Sekunden und in einer ganz bevorzugten Form bei 20 bis 60 Sekunden liegt.

46. Verfahren nach einem der Ansprüche 44 oder 45, **dadurch gekennzeichnet, daß** zwischen 0,1 µl und 500 µl, vorzugsweise zwischen 1 µl und 100 µl und am bevorzugtesten Mengen von 10 µl bis 30 µl Lösung mit reaktionsspezifischem Substrat aufpipettiert werden.

47. Verfahren nach einem der Ansprüche 44 bis 46, **dadurch gekennzeichnet, daß** die Inkubationszeit in einem Bereich von 2 bis 100 Sekunden, vorzugsweise von 5 bis 30 Sekunden und am bevorzugtesten bei 20 Sekunden liegt.

48. Verfahren nach einem der Ansprüche 44 bis 47, **dadurch gekennzeichnet, daß** das abgelesene elektrische Signal mit einem weiteren elektrischen Signal verglichen wird, welches von einer zweiten, unspezifischen Arbeitselektrode **(40")** stammt, wobei diese zweite Arbeitselektrode **(40")** nur mit reaktionsunspezifischen Liganden oder Bindungsmolekülen versehen ist.

49. Verwendung einer Vorrichtung **(10)** nach einem der Ansprüche 1 bis 38 und eines Verfahrens insbesondere nach einem der Ansprüche 44 bis 48 zum Erfassen sowie zur Bestimmung der Konzentration wenigstens eines Analyten aus Körperflüssigkeiten wie Vollblut, Plasma, Serum, Urin, Sekret, Liquor sowie aus Extrakten von Körpergeweben und aus Abstrichmaterial.

## Claims

1. Device fort he detection and/or determination of the concentration of at least one analyte existing in a liquid medium, and consisting
a) of a strip-shaped support **(20)** with two longitudinal edges **(24),** on which is applied a printed electrode set consisting of at least one reference electrode **(30)** and of at least one working electrode **(40),** [and]
with each working electrode **(40)** and each reference electrode **(30)** being conductively connected to a strip conductor **(42)** assigned to it and each strip conductor **(42)** to a contact **(44)** assigned to it and being connectible to a an electric meter,
b) of ligands suitable to bind a biologically active specific bonding molecule or the analyte
c) and of a sample application zone **(22)** formed at one end of the support **(20),**
**characterized by**
a) the device possessing a rigid reaction- and detection space **(50)** for continuous flow-through along the strip-shaped support **(20)** and being formed of a longish rest **(52)** with a covering **(54)** arranged on its top and positioned on the longitudinal edges (24) of the support **(20),**
b) a liquid receiver element **(60)** being arranged downstream the reaction- and detection space **(50),**
c) the longish rest **(52)** being bipartite and in the form of two stripes resting in permanent adhesion on the two longitudinal edges **(24)** of the support **(20)** and
d) the strips resting only on a part of the two longitudinal edges **(24)** of the support **(20),** so that between the stripes and the liquid reception receiver element **(60)** a gap **(70)** exists, from where the stripes stretch upstream on the longitudinal edges **(24).**

2. Device according to claim 1, **characterized by** the support **(20)** is designed as a self-supporting flow surface for the liquid medium.

3. Device according to claim 1 or 2, **characterized by** the support **(20)** being single-piece and consisting of a polymeric, non-conducting, preferably rectangular material.

4. Device according to one of the claims 1 to 3, **characterized by** ligands and/or specific biologically active bonding molecules are applied.

5. Device according to one of the claims 1 to 3, **characterized by** the ligands and/or the biologically active specific bonding molecules on the support **(20)** are immobilized.

6. Device according to one of the claims 1 to 5, **characterized by** the support **(20)** being equipped with a basic coating consisting of proteins of the group of animal or human albumins and/or of the group of globulins and/or of the group of glycoproteins.

7. Device according to one of the claims 1 to 6, **characterized by** the support **(20)** having a basic coating consisting of polypeptides, for example poly-amino acids and/or degradation products of proteins preferably of the supporting and connective tissue.

8. Device according to one of the claims 1 to 7, **characterized by** the sample application zone **(22)** or at least a part of it is treated with surface-active substances and has the ability to absorb the liquid medium.

9. Device according to one of the claims 1 to 8, **characterized by** a mesh-structured or porous material treated preferably with surface-active substances being applied on the sample application zone **(22).**

10. Device according to one of the claims 1 to 10, **characterized by** at least one of the electrodes **(30, 40)** as well as the respective strip conductor **(42)** assigned to it and the respective contact **(44)** assigned to it consisting of gold, silver, platinum, nickel, palladium, titanium, copper or carbon.

11. Device according to one of the claims 1 to 10, **characterized by** at least one of the electrodes **(30, 40)** consisting of a dried graphite paste, or being an electrode that can be made of metal pastes and/or metallic salt pastes.

12. Device according to one of the claims 1 to 11, **characterized by** at least one working electrode **(40)** carried ligands and/or biologically active specific bonding molecules, for example on its surface.

13. Device according to one of the claims 1 to 12, **characterized by** on at least one other working electrode (40) reaction non-specific bonding molecules are arranged and/or immobilized.

14. Device according to one of the claims 1 to 13, **characterized by** the ligands being applied as a solution of a concentration of 0,0007 to 0,7 mol/l, preferably of 0,035 to 0,35 mol/l.

15. Device according to one of the claims 1 to 14, **characterized by** the ligands being made of ribonucleic-acids or deoxyribonucleic acids.

16. Device according to one of the claims 1 to 15, **characterized by** the ligands consisting of amino acids, preferably of diamino-carbon acid.

17. Device according to claim 16, **characterized by** the ligands consisting of polymolecular homogenous or heterogenous peptide-like linked amino acids of the composition R-CH(NH₂)-(CH₂)ₙ-COOH, for example of poly-amino acids where the residue R stands for a proton or preferably for an amino-, imino-, hydroxyl-, thiol-, hydroxy-alkyl-, amino alkyl-, or carboxy-alkyl-group and n becomes a value of 0 to 6, preferably 0.

18. Device according to one of the claims 1 to 17, **characterized by** the ligands consisting of proteides respectively of proteins that preferably stem from the bacterium streptomyces avidinii.

19. Device according to one of the claims 1 to 18, **characterized by** at least one of the ligands being a phenylboric acid to detect HbA1c.

20. Device according to one of the claims 1 to 19, **characterized by** the biologically active specific bonding molecules having bonding sites for at least one ligand or are bound to it.

21. Device according to one of the claims 1 to 20, **characterized by** the biologically active specific bonding molecules having at least one specific bonding region to bond one analyte.

22. Device according to one of the claims 1 to 21, **characterized by** one catalytically active protein being preferably covalently bonded to the biologically active specific bonding molecules and/or the ligands or more catalytically active proteins being preferably covalently bonded to the biologically active specific bonding molecules and/or ligands.

23. Device according to one of the claims 1 to 22, **characterized by** the stripes consisting of a polymer material and/or a hot-setting adhesive and being of the same thickness as the liquid receiver element (60).

24. Device according to one of the claims 1 to 23, **characterized by** the covering **(54)** consisting of a polymeric, preferably rectangular material and being connected with the longish rest (52) such that it covers the support (20) as well as the multiple electrode set and the liquid receiver element (60).

25. Device according to one of the claims 1 to 24, **characterized by** the covering **(54)** not covering but one or more parts of the support (20).

26. Device according to one of the claims 1 to 25, **characterized by** the covering (54) featuring at its upstream side at least one slot-shaped and/or semicircular respectively polygonal recess (56) and/or aperture (56) arranged in a way that the sample application zone (22) of the support (20) is chargeable with liquid medium.

27. Device according to one of the claims 1 to 26, **characterized by** the liquid receiver element (60) is adhesively fixed to a side of the covering (54) that faces the support (20).

28. Device according to one of the claims 1 to 27, **characterized by** the covering **(54)** or at least one part of it being treated with surface-active substances.

29. Device according to one of the claims 1 to 28, **characterized by** the side of the covering **(54)** facing the support **(20)** having in the region of the multiple electrode set, especially above the working electrode(s) **(40)** and above the reference electrode **(30)** hydrophilic surface properties and showing at the site of the sample application zone **(22)** at least in partly a hydrophobic surface.

30. Device according to one of the claims 1 to 29, **characterized by** the reaction- and detection space **(50)** being open in downstream and upstream direction and the gap **(70)** being downstream adjacent to it.

31. Device according to one of the claims 1 to 30, **characterized by** the liquid receiver element **(60)** on the support **(20)** being arranged in downstream distance from the from the sample application zone **(22)** and covering the strip conductors **(42),** for example by resting fixedly on them.

32. Device according to one of the claims 1 to 31, **characterized by** the liquid receiver element **(60)** consisting of an absorptive porous fibrillary material and being adhesively fixed to the support **(20).**

33. Device according to one of the claims 1 to 32, **characterized by** the porous material consisting of at least one fibre layer or of at least one tissue consisting of cellulose fibres or glass fibres or mixtures thereof, respectively being a composition of organic polymers.

34. Device according to one of the claims 1 to 33, **characterized by** the liquid receiver element **(60)** being treated with surface-active substances, especially such of hydrophilic properties.

35. Device according to one of the claims 8 to 34, **characterized by** the surface-active substances containing hydrophilic groups, - for example COOMe, -OSO₃Me, -SO₃Me, -NH₂, =NH, -NR₃+, and hydrophobic alkyl chains of 10 to 18 c-atoms, or alkyl aryl residues.

36. Device according to one of the claims 8 to 35, **characterized by** agents of the empirical formulas C20H37NaO7S or C₁₈H₂₉NO₃S being preferably used as surface-active substances.

37. Device according to one of the claims 3 to 36, **characterized by** the polymeric material being a polyethylene, a polystyrene, a polyurethane, a polyvinyl acetate, a polyester, for example polyethylene terephthalate, an epoxy resin, a methacrylic polymer, a polycarbonate, a polyvinyl chloride, or a copolymer of the named compounds.

38. Device according to one of the claims 3 to 37, **characterized by** the polymeric material being a film preferably made of polyester, polycarbonate, or polyvinyl chloride.

39. Method to produce a device **(10)** for the detection and determination of the concentration of at least one analyte dissolved in a liquid medium according to one of the claims 1 to 38, **characterized by**
a) an electrode material being worked to a paste with this paste being applied to a support **(20)** such that a multiple electrode set of at least one working electrode **(40)** and at least one reference electrode **(30)** as well as of thereto connected strip conductors **(42)** and contacts **(44)** is formed,
b) the support **(20)** equipped with the multiple electrode set is being dried at raised temperature,
c) ligands and/or biologically active specific bonding molecules and/or catalytically active proteins being applied on the support **(20),** namely in the region of the multiple electrode set or in a region arranged upstream the multiple electrode set, and
d) a longish rest **(52),** a covering **(54)** and a liquid receiver element **(60)** being fixed on the such obtained support (20) in a way that the covering **(54)** and the support **(20)** are in a distance to each other.

40. Method according to claim 39, **characterized by** the paste containing ligands and/or biologically active specific bonding molecules and/or catalytically active proteins and being applied by silk-screen technique.

41. Method according to one of the claims 39 or 40, **characterized by** at least one of the dried electrodes **(40)** being coated with ligands and/or biologically active specific bonding molecules and/or catalytically active proteins preferably by using cross-linking molecules or coupling reagents.

42. Method according to one of the claims 39 to 41, **characterized by** at least one of the dried electrodes **(40)** being coated with ligands and/or reaction non-specific bonding molecules preferably by using cross-linking molecules or coupling reagents.

43. Method according to one of the claims 39 to 42, **characterized by** the liquid receiver element **(60)** being treated and dried with the solution of a surface-active substance that in its preferred type is a compound of the empirical formula C₂₀H₃₇NaO 7S or C18H₂₉NO₃S and being used at a concentration of 0,001 to 5 % w/v, preferably from 0,1 to 3 % w/v.

44. Method for the detection and the determination of the concentration of at least one analyte dissolved in a liquid medium with a device **(10)** consisting of at least one working electrode **(40)** and at least one reference electrode **(30)** according to one of the claims 1 to 43, **characterized by**
a) a definable amount of the liquid medium being applied to a sample application zone **(22)** of the device **(10),**
b) a given waiting time period for an interaction between the analyte/s with ligands and/or biologically active specific bonding molecules and/or catalytically active proteins to take place,
c) application being made to the sample application zone **(22)** of a solution containing at least one reaction-specific substrate for one catalytically active protein or for more catalytically active proteins,
d) the device **(10)** being connected to an electric meter, and
e) an electric signal for each analyte to be determined being read from the meter connected to the device **(10)** after an incubation time, and the corresponding concentration of the respective analyte being assigned to such signal by means of a calibration curve.

45. Method according to claim 44, **characterized by** the definable quantity being of 0,01 to 20 µ, preferably 1 to 10 µ, and the given time lying between 5 and 600 seconds, preferably between 10 and 120 seconds and in the most preferably form at 20 to 60 seconds.

46. Method according to one of the claims 44 or 45, **characterized by** between 0,1 µ and 500 µ, preferably between 1 µ and 100 µ, and most preferably quantities of 10 µ to 30 µ solution of reaction-specific substrate being pipetted.

47. Method according to one of the claims 44 to 46, **characterized by** the incubation time lying within a period of 2 to 100 seconds, preferably of 5 to 30 seconds, and most preferably at 20 seconds.

48. Method according to one of the claims 44 to 47, **characterized by** the read electric signal being compared with an additional electric signal originating from a second unspecific working electrode **(40"),** with this second working electrode **(40")** being equipped only with reaction non-specific ligands or bonding molecules.

49. Use of a device **(10)** accordingb to one of the claims 1 to 38 and of a method especially according to one of the claims 44 to 48 for the detection and determination of at least one analyte from body fluids such as full blood, plasma, serum, urine, secretion, liquor, as well as from body tissue extracts and from swab material.

## Revendications

1. Dispositif destiné à enregistrer et/ou déterminer la concentration d'au moins un analyte présent dans un liquide, comprenant
a) un porteur **(20)** en forme de bande, comprenant deux bords longitudinaux **(24),** sur lequel est appliqué un jeu d'électrodes multiples imprimées, constitué d'au moins une électrode de référence **(30)** et d'au moins une électrode de travail **(40),** [et]
chaque électrode de travail **(40)** et chaque électrode de référence **(30)** étant raccordées de manière conductrice à un circuit conducteur **(42)** lui étant affecté et chaque circuit conducteur **(42)** étant raccordé de manière conductrice à un contact **(44)** lui étant affecté, raccordable à un appareil de mesure électrique,
b) des ligands aptes à lier une molécule de liaison spécifique, biologiquement active, ou l'analyte
c) et une zone d'application d'échantillons **(22)** formée sur une extrémité du porteur **(20),**
**caractérisé en ce**
a) **que** le dispositif possède une chambre de réaction et de détection **(50)** rigide, pouvant être traversée de manière continue le long du porteur **(20)** en forme de bande, cette chambre de réaction et de détection étant formée par un support **(52)** allongé, posé sur les bords longitudinaux **(24)** du porteur **(20),** muni d'un recouvrement **(54)** disposé sur le support,
b) en ce qu'un élément d'absorption de liquide **(60)** est disposé en aval de la chambre de réaction et de détection **(50),**
c) en ce que le support allongé **(52)** est divisé en deux et repose sur les deux bords longitudinaux **(24)** du porteur **(20)** sous forme de deux bandes, en étant adhérent en permanence et
d) en ce que les bandes reposent seulement sur une partie des deux bords longitudinaux **(24)** du porteur **(20),** un espace intermédiaire **(70)** étant présent entre les bandes et l'élément d'absorption de liquide **(60),** duquel s'étendent les bandes en amont sur les bords longitudinaux **(24).**

2. Dispositif selon la revendication 1, **caractérisé en ce que** le porteur **(20)** est exécuté en tant que surface d'écoulement autoporteuse pour le liquide.

3. Dispositif selon la revendication 1 ou 2, **caractérisé en ce que** le porteur **(20)** est exécuté d'une seule pièce et constitué d'une matière polymère non-conductrice, de préférence rectangulaire.

4. Dispositif selon l'une quelconque des revendications 1 à 3, **caractérisé en ce que** des ligands et/ou des molécules de liaison spécifiques, biologiquement actives, sont appliqués sur le porteur **(20).**

5. Dispositif selon la revendication 4, **caractérisé en ce que** les ligands et/ou les molécules de liaison spécifiques, biologiquement actives, sont immobilisés sur le porteur **(20).**

6. Dispositif selon l'une quelconque des revendications 1 à 5, **caractérisé en ce que** le porteur **(20)** est équipé d'une couche de base constituée de protéines du groupement des albumines d'origine animale ou humaine et/ou du groupement des globulines et/ou du groupement des glycoprotéines.

7. Dispositif selon l'une quelconque des revendications 1 à 6, **caractérisé en ce que** le porteur **(20)** est muni d'une couche de base constituée de polypeptides, par exemple de polyaminoacides, et/ou de produits cataboliques de protéines, de préférence du tissu de soutien et du tissu conjonctif.

8. Dispositif selon l'une quelconque des revendications 1 à 7, **caractérisé en ce que** la zone d'application d'échantillons **(22)** ou au moins une partie d'elle est traitée avec des substances tensioactives et possède le pouvoir d'absorber le liquide.

9. Dispositif selon l'une quelconque des revendications 1 à 8, **caractérisé en ce qu'**une matière en forme de mailles ou poreuse, traitée de préférence avec des substances tensioactives, est appliquée sur la zone d'application d'échantillons **(22).**

10. Dispositif selon l'une quelconque des revendications 1 à 9, **caractérisé en ce qu'**au moins une des électrodes (30, 40) ainsi que le circuit conducteur (42) qui lui est respectivement affecté et le contact (44) qui lui est respectivement affecté sont constitués d'or, d'argent, de platine, de nickel, de palladium, de titan, de cuivre ou de carbone.

11. Dispositif selon l'une quelconque des revendications 1 à 10, **caractérisé en ce qu'**au moins une des électrodes (30, 40) est constituée d'une pâte de graphite séchée ou est une électrode pouvant être fabriquée par séchage à partir de pâtes métalliques et/ou de pâtes au sel métallique.

12. Dispositif selon l'une quelconque des revendications 1 à 11, **caractérisé en ce qu'**au moins une électrode de travail (40) porte, par exemple sur sa surface, des ligands et/ou des molécules de liaison spécifiques, biologiquement actives.

13. Dispositif selon l'une quelconque des revendications 1 à 12, **caractérisé en ce que** des molécules de liaison non spécifiques à la réaction sont disposées et/ou immobilisées sur au moins une autre électrode de travail (40).

14. Dispositif selon l'une quelconque des revendications 1 à 13, **caractérisé en ce que** les ligands sont appliqués en tant que solution ayant une concentration de 0,0007 à 0,7 mol/l, de préférence de 0,035 à 0,35 mol/l.

15. Dispositif selon l'une quelconque des revendications 1 à 14, **caractérisé en ce que** les ligands sont formés à partir d'acides ribonucléiques ou d'acides désoxyribonucléiques.

16. Dispositif selon l'une quelconque des revendications 1 à 15, **caractérisé en ce que** les ligands sont constitués d'aminoacides, de préférence de diaminoacides.

17. Dispositif selon la revendication 16, **caractérisé en ce que** les ligands sont constitués d'aminoacides polymoléculaires, liés similairement à des peptides de manière homogène ou hétérogène, de la composition R-CH(NH₂)-(CH₂)ₙ-COOH, par exemple de polyaminoacides, le reste R étant un proton ou de préférence un groupement aminé, un groupement imine, un groupement hydroxyle, un groupement thiol, un groupement hydroxyle, un groupement aminoalkyle ou un groupement carboxyle-alkyle et n prenant de préférence une valeur de 0 à 6, de préférence 0.

18. Dispositif selon l'une quelconque des revendications 1 à 17, **caractérisé en ce que** les ligands sont constitués de protéides ou de protéines provenant de préférence du bacterium Streptomyces avidinii.

19. Dispositif selon l'une quelconque des revendications 1 à 18, **caractérisé en ce que**, par exemple pour la détection de HbA1c, au moins un ligand est un acide phényle.

20. Dispositif selon l'une quelconque des revendications 1 à 19, **caractérisé en ce que** les molécules de liaison spécifiques, biologiquement actives, possèdent des endroits de liaison pour au moins un ligand ou sont reliées à lui.

21. Dispositif selon l'une quelconque des revendications 1 à 20, **caractérisé en ce que** les molécules de liaison spécifiques, biologiquement actives, présentent au moins une région de liaison spécifique pour la liaison d'un analyte.

22. Dispositif selon l'une quelconque des revendications 1 à 21, **caractérisé en ce qu'**une protéine catalytiquement active est liée, de préférence de manière covalente, aux molécules de liaison spécifiques, biologiquement actives, et/ou aux ligands ou **en ce que** plusieurs protéines catalytiquement actives sont liées, de préférence de manière covalente, aux molécules de liaison spécifiques, biologiquement actives, et/ou aux ligands.

23. Dispositif selon l'une quelconque des revendications 1 à 22, **caractérisé en ce que** les bandes sont constituées d'une matière polymère et/ou d'une colle adhésive et possèdent la même épaisseur que l'élément d'absorption de liquide **(60).**

24. Dispositif selon l'une quelconque des revendications 1 à 23, **caractérisé en ce que** le recouvrement **(54)** est constitué d'une matière polymère, de préférence rectangulaire, et est raccordé au support allongé (52) de telle manière qu'il recouvre le porteur **(20)** ainsi que le jeu d'électrodes multiples et l'élément d'absorption de liquide **(60).**

25. Dispositif selon l'une quelconque des revendications 1 à 24, **caractérisé en ce que** le recouvrement **(54)** recouvre seulement une ou plusieurs parties du porteur **(20).**

26. Dispositif selon l'une quelconque des revendications 1 à 25, **caractérisé en ce que** le recouvrement **(54),** sur son côté en amont, possède au moins un évidement **(56)** en forme de fente et/ou de demi-cercle ou polygonal, et/ou une ouverture **(56)** qui est disposée de telle manière que la zone d'application d'échantillons **(22)** du porteur **(20)** peut être alimentée avec du liquide.

27. Dispositif selon l'une quelconque des revendications 1 à 26, **caractérisé en ce que** l'élément d'absorption de liquide **(60)** est fixé de manière adhérente sur un côté, tourné vers le porteur **(20),** du recouvrement **(54).**

28. Dispositif selon l'une quelconque des revendications 1 à 27, **caractérisé en ce que** le recouvrement **(54)** ou au moins une partie de lui est traité avec des substances tensioactives.

29. Dispositif selon l'une quelconque des revendications 1 à 28, **caractérisé en ce que** le côté, tourné vers le porteur **(20),** du recouvrement **(54)** présente des caractéristiques superficielles hydrophiles dans la partie du jeu d'électrodes multiples, notamment au-dessus de l'électrode (des électrodes) de travail **(40)** et au-dessus de l'électrode de référence **(30),** et présente au moins en partie une surface hydrophobe à l'endroit de la zone d'application d'échantillons **(22).**

30. Dispositif selon l'une quelconque des revendications 1 à 29, **caractérisé en ce que** la chambre de réaction et de détection **(50)** est ouverte en direction en aval et en direction en amont et **en ce que** l'espace intermédiaire **(70)** lui est adjacent en aval.

31. Dispositif selon l'une quelconque des revendications 1 à 30, **caractérisé en ce que** l'élément d'absorption de liquide (60) est disposé sur le porteur **(20)** en aval en étant à distance de la zone d'application d'échantillons **(22)** et recouvre les circuits conducteurs **(42),** par exemple repose de manière fixe sur eux.

32. Dispositif selon l'une quelconque des revendications 1 à 31, **caractérisé en ce que** l'élément d'absorption de liquide **(60)** est constitué d'une matière fibrillaire poreuse absorbante et est fixé sur le porteur **(20)** de manière adhérente.

33. Dispositif selon la revendication 32, **caractérisé en ce que** la matière poreuse est composée d'au moins une couche de fibres ou d'au moins un tissu, laquelle/lequel est constitué(e) de fibres de cellulose ou de fibres de verre ou de mélanges de celles-ci ou se compose de polymères organiques.

34. Dispositif selon l'une quelconque des revendications 1 à 33, **caractérisé en ce que** l'élément d'absorption de liquide **(60)** est traité avec des substances tensioactives, de préférence avec de telles substances tensioactives ayant des propriétés hydrophiles.

35. Dispositif selon l'une quelconque des revendications 8 à 34, **caractérisé en ce que** les substances tensioactives comprennent des groupes hydrophiles, par exemple COOMe, -OSO₃Me,- SO₃Me, -NH₂, = NH, -NR₃⁺ et des chaînes alkyles hydrophobes de 10 à 18 atomes C ou des alkylaryles.

36. Dispositif selon l'une quelconque des revendications 8 à 35, **caractérisé en ce que** l'on utilise de préférence des substances ayant les formules brutes C₂₀H₃₇NaO₇S ou C₁₈H₂₉NO₃S en tant que substances tensioactives.

37. Dispositif selon l'une quelconque des revendications 3 à 36, **caractérisé en ce que** la matière polymère est un polyéthylène, un polystyrène, un polyuréthanne, un polyacétate de vinyle, un polyester, par exemple un polytéréphtalate, une résine époxyde, un polymère méthacrylique, un polycarbonate, un chlorure de polyvinyle ou un copolymère des liaisons mentionnées.

38. Dispositif selon l'une quelconque des revendications 3 à 37, **caractérisé en ce que** la matière polymère est une feuille fabriquée de préférence en polyester, polycarbonate ou chlorure de polyvinyle.

39. Procédé de fabrication d'un dispositif **(10)** destiné à enregistrer et/ou déterminer la concentration d'au moins un analyte dissous dans un liquide, selon l'une quelconque des revendications 1 à 38, **caractérisé en ce**
a) **qu'**une matière d'électrode est transformée en une pâte et en ce que cette pâte est appliquée sur un porteur **(20)** de telle manière qu'un jeu d'électrodes multiples imprimées, constitué d'au moins une électrode de référence **(30)** et d'au moins une électrode de travail **(40)** ainsi que de circuits conducteurs **(42)** et de contacts **(44)** qui y sont raccordés, est formé,
b) en ce que le porteur **(20)** muni du jeu d'électrodes multiples est séché à une température élevée,
c) en ce que des ligands et/ou des molécules de liaison spécifiques, biologiquement actives, et/ou des protéines catalytiquement actives sont appliqués sur le porteur **(20),** à savoir dans la partie du jeu d'électrodes multiples ou dans une partie disposée en amont du jeu d'électrodes multiples, et
d) en ce que sur le porteur **(20)** ainsi obtenu, un support allongé **(52),** un recouvrement **(54)** et un élément d'absorption de liquide **(60)** sont fixés de telle manière que le recouvrement **(54)** et le porteur **(20)** ont un écart l'un par rapport à l'autre.

40. Procédé selon la revendication 39, **caractérisé en ce que** la pâte comprend des ligands et/ou des molécules de liaison spécifiques, biologiquement actives, et/ou des protéines catalytiquement actives et est appliquée par sérigraphie.

41. Procédé selon l'une quelconque des revendications 39 ou 40, **caractérisé en ce qu'**au moins une des électrodes **(40)** séchées est enduite de ligands et/ou de molécules de liaison spécifiques, biologiquement actives, et/ou de protéines catalytiquement actives, de préférence en utilisant des molécules de réticulation ou des réactifs de copulation.

42. Procédé selon l'une quelconque des revendications 39 à 41, **caractérisé en ce qu'**au moins une des électrodes **(40)** séchées est enduite de ligands et/ou de molécules de liaison non spécifiques à la réaction, de préférence en utilisant des molécules de réticulation ou des réactifs de copulation.

43. Procédé selon l'une quelconque des revendications 39 à 42, **caractérisé en ce que** l'élément d'absorption de liquide **(60)** est traité avec une solution d'une substance tensioactive et est séché, cette substance étant, dans une exécution préférée, une liaison de la formule brute C₂₀H₃₇NaO₇S ou C₁₈H₂₉NO₃S et étant utilisée dans une concentration de 0,001 à 5 % poids/volume, de préférence de 0,1 à 3% poids/volume.

44. Procédé destiné à enregistrer et/ou déterminer la concentration d'au moins un analyte dissous dans un liquide, au moyen d'un dispositif (10) constitué d'au moins une électrode de travail **(40)** et d'au moins une électrode de référence **(30),** selon l'une quelconque des revendications 1 à 43, **caractérisé en ce**
a) **qu'**une quantité définissable du liquide est appliquée sur une zone d'application d'échantillons **(22)** du dispositif **(10),**
b) en ce qu'un temps prédéfinissable est attendu, afin qu'une interaction de l'analyte/des analytes avec les ligands et/ou les molécules de liaison spécifiques, biologiquement actives, et/ou les protéines catalytiquement actives puisse avoir lieu,
c) en ce qu'une solution est appliquée sur la zone d'application d'échantillons **(22),** cette solution comprenant au moins un substrat, spécifique à la réaction, pour une protéine catalytiquement active ou pour plusieurs protéines catalytiquement actives,
d) en ce que le dispositif **(10)** est raccordé à un dispositif de mesure électrique et
e) en ce qu'après un temps d'incubation, un signal électrique est lu sur l'appareil de mesure raccordé au dispositif **(10)** pour chaque analyte à déterminer et en ce qu'une concentration correspondante de l'analyte respectif est associée à ce signal à l'aide d'une courbe d'étalonnage.

45. Procédé selon la revendication 44, **caractérisé en ce que** la quantité définissable est de 0,01 à 20 µl, de préférence de 1 à 10µl, et **en ce que** le temps prédéfinissable est compris entre 5 et 600 secondes, de préférence entre 10 et 120 secondes, et que, dans une forme tout à fait préférée, il est de 20 à 60 secondes.

46. Procédé selon l'une quelconque des revendications 44 ou 45, **caractérisé en ce que** des quantités de solution, comprenant un substrat spécifique à la réaction, comprises entre 0,1 µl et 500µl, de préférence entre 1µl et 100 µl et de manière la plus préférée des quantités de 10 µl à 30µl sont pipetées.

47. Procédé selon l'une quelconque des revendications 44 à 46, **caractérisé en ce que** le temps d'incubation est compris dans une plage de 2 à 100 secondes, de préférence de 5 à 30 secondes et que de manière la plus préférée, il est de 20 secondes.

48. Procédé selon l'une quelconque des revendications 44 à 47, **caractérisé en ce que** le signal électrique lu est comparé à un autre signal électrique, lequel provient d'une deuxième électrode de travail **(40")** non spécifique, cette deuxième électrode de travail **(40")** étant munie seulement de ligands ou de molécules de liaison non spécifiques à la réaction.

49. Utilisation d'un dispositif **(10)** selon l'une quelconque des revendications 1 à 38 et d'un dispositif, notamment selon l'une quelconque des revendications 44 à 48, destinés à enregistrer et/ou déterminer la concentration d'au moins un analyte constitué de liquides corporels, tels que sang complet, plasma, sérum, urine, substances sécrétées, liquide céphalo-rachidien, ainsi que d'extraits de tissus tissulaires et de matières de prélèvements.
